# EUROPEAN PATENT SPECIFICATION

(11) **EP 0 426 195 B1**
(45) Date of publication and mention of the grant of the patent: **17.10.2001**
(21) Application number: 90121052.6
(22) Date of filing: 02.11.1990
(51) Int. Cl.: C12N 15/40, C12N 15/82, C12N 15/11, A01H 5/00, C12Q 1/68

(54) **Improvements in or relating to organic compounds**
Verbesserungen von oder in bezug auf organische Verbindungen
Améliorations concernant des compositions organiques

(30) Priority: 03.11.1989 US 431259; 05.12.1989 US 446024
(43) Date of publication of application: 08.05.1991
(73) Proprietor: Novartis Seeds B.V., 1601 BK Enkhuizen (NL)
(72) Inventor: Goldbach, Robert Willem, NL-6721 DA Bennekom (NL); Peters, Dirk, NL-6703 Wageningen (NL); Gielen, Johannes Jacobus Ludgerus, NL-1602 GC Enkhuizen (NL); De Haan, Petrus Theodorus, NL-6668 AH Randwijk (NL); Kool, Arnoldus Johannes, NL-1602 LJ Enkhuizen (NL); Van Grinsven, Martinus Quirinus Joseph Marie, NL-1602 MA Enkhuizen (NL)
(74) Representative: Becker, Konrad

(56) References cited:
- EP-A- 0 223 452
- EP-A- 0 240 332
- BIOLOGICAL ABSTRACTS, BR36:106479, Philadelphia, PA, US; T.L. GERMAN et al.: "Dot blot detection of tomato spotted wilt virus using cloned complementary DNA probes", & PHYTOPATHOLOGY, 78, (12 part 1), 1988, 1599
- BIOLOGICAL ABSTRACTS, vol. 89, no. 5, 1990, abstract no. 52485, Philadelphia, PA, US; A.E.E. RONCO et al.: "Cloned complementary DNA probes for the detection of tomato spotted wilt virus", & PHYTOPATHOLOGY 79(11): 1309-1313, 1989
- J. GEN. VIROL., vol. 70, no. 12, December 1989, pages 3469-3473, SGM, GB; P. DE HAAN et al.: "Molecular cloning and terminal sequence determination of the S and M RNAs of tomato spotted wilt virus"
- J. GEN. VIROL., vol. 71, no. 5, May 1990, pages 1001-1007, SGM, GB; P. DE HAAN et al.: "The S RNA segment of tomato spotted wilt virus has an ambisense character"
- BIOLOGICAL ABSTRACTS, vol. 89, no. 11, 1990, abstract no. 121862, Philadelphia, PA, US; C. HUGUENOT et al.: "Detection of tomato spotted wilt virus using monoclonal antibodies and riboprobes", & ARCH. VIROL. 110(1/2): 47-62

## Description

### Scope of the invention

The present invention relates to plants having reduced susceptibility to infection with tospoviruses, genetic material used to generate this tolerance, probes for the isolation or diagnosis and processes for obtaining such plants and genetic material and probes.

### Introduction

In general, virus infections have a variety of detrimental effects on e.g. growth, morphology and yield of plants. Also, virus infections often result in higher susceptibility of infected plants to other plant pathogens and plant pests. Transmission of plant viruses occurs generally by insect or fungal vectors or mechanically.

Plant breeders are trying continuously to develop varieties of crop plant species tolerant to or resistant to specific virus strains. Traditionally virus resistance genes are transferred from wild relatives into the commercial varieties by breeding. The transfer of an existing resistance from the gene pool of wild relatives to a cultivar is a tedious process in which the resistance gene(s) first have to be identified in a source (donor) plant species and the resistance genes have to be combined with the perfect gene pool of a commercial variety. Resistances or tolerances generated in this way are in many cases only active against one or a few strains of the virus in question. Also, breeding cultivars for resistance to a particular virus species is often limited by a lack of genetic sources for this resistance within the crop species. Other approaches to prevent or decrease the effect of virus disease on plants are the use of chemicals or other methods which act against the virus vectors such as e.g. the use of insecticides, fungicides or good phytosanitary working conditions. However, the use of chemicals to combat virus disease by killing the vector is subject to increasing stricter regulations for use imposed by governments, confronting the growers with a decreasing scala of allowed chemical plant-protectants.

Alternatively, a system called "cross-protection" can be employed. Cross-protection is a phenomenon in which infection of a plant with one strain of a virus protects the plant against superinfection with a second related virus strain. Thus, this latter method preferentially involves the use of avirulent virus strains to infect plants, to inhibit a secondary infection with a virulent strain of the same virus. However, the use of this natural cross-protection system has several disadvantages. The method is very laborious because it requires inoculation of every planted crop, and carries the risk that due to a mutation the former avirulent strain becomes a more virulent strain, thereby creating a disease by itself. It is also possible that an avirulent virus strain on one plant species acts as a virulent strain on another plant species.

T. L. German et al. Phytopathology, 78, (12 part 1), 1988, 1599; is an abstract of an oral presentation and discloses dot blot detection of TSWV in plants (no technical teaching in abstract).

Several studies indicated that the viral coat protein of the protecting virus plays an important role in the cross-protection and that protection occurs when the resident virus and the challenging virus have the same or a closely related coat protein structures.

With the recent development of genetic manipulation and plant transformation systems new methods to create virus resistance have emerged. Genetically engineered cross-protection is a form of virus resistance which phenotypically resembles the natural cross-protection, but is achieved through expression of the genetic information of the viral coat protein from the genome of a genetically manipulated plant. The first successful experiments generating virus resistance by genetic engineering were performed by Beachy *et al*. (1985) and Abel *et al*. (1986). They showed that expression of the tobacco mosaic virus strain U1 (TMV-U1) coat protein gene from the genome of a transgenic plant resulted in a delay of symptom development after infection with any TMV strain. Similar results with respect to coat protein-mediated protection have been obtained for alfalfa mosaic virus (AMV), potato virus X (PVX) and cucumber mosaic virus (CMV).

Also EP-A-0 223 452 addresses the general problem of making plants resistant to viral infection by inserting into the genome of a plant cell viral coat protein.

Although TMV, CMV, AMV and PVX belong to different virus groups, they share a a common architecture: in all these virions the viral RNA is a positive strand RNA encapsidated by a viral coat consisting of many individual but identical viral coat proteins.

Tospoviruses are essentially different from these plant virions. The genus of tospoviruses belongs to the Bunyaviridae. All tospoviruses are thrips transmitted. The virus particles are spherically shaped (80-120 nm in diameter) and contain internal nucleocapsids surrounded by a lipid envelope studded with glycoprotein surface projections. The multipartite genome consists of linear single stranded RNA molecules of negative or ambisense polarity. The terminal nucleotides of these RNA molecules are characterised by a consensus sequence as follows: 5' AGAGCAAUX....................GAUUGCUCU 3', wherein X is C or U. Typical members of the tospoviruses are tomato spotted wilt virus (TSWV) and Impatiens necrotic spot virus, also known as TSWV eyetype.

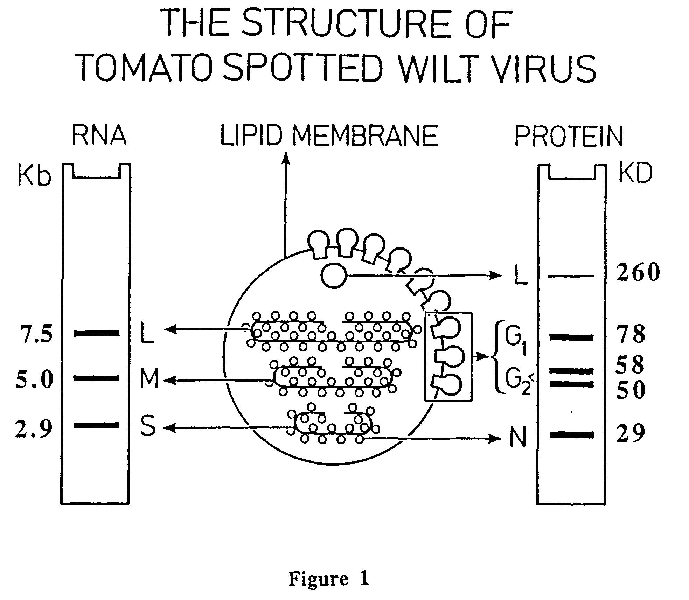

The TSWV virion contains 4 distinct structural proteins: an internal nucleocapsid protein N of 29 kd and two membrane glycoproteins: G₁ of approximately 78 kd and G₂ of approximately 58 kd. In addition, minor amounts of a large protein L of approximately 260 kd have been detected in virus particles. The genome of TSWV consists of three linear single stranded RNA molecules of ±2900 nt (S RNA), ±5000 nt (M RNA) and ±7500 nt (L RNA), each tightly associated with nucleocapsid proteins and a few copies of the L protein to form circular nucleocapsids. A schematic structure outlining most properties of the TSWV virion is given in figure 1. Based on these and other properties TSWV has been classified as a representative of the tospoviruses group. Moreover, TSWV is considered as the typemember of the tospoviruses. Only circumstantial evidence was presented that suggested that a M RNA encoded gene is directly or indirectly involved in the synthesis of the G₁ membrane glycoprotein (Verkleij and Peters, 1983). The coding properties of the other RNA molecules and the polarity of the genomic RNA were still unknown prior to this invention.

As stated earlier, tospoviruses such as TSWV are transmitted by certain thrips species. The vectors of TSWV belong to the family of *Tripidae* and include tobacco thrips (*Frankliniella fusca* (Hinds.)), western flower thrips (*F. occidentalis* (Pergande)), common blossom thrips *(F. Schultzei* (Trybom)), chilli thrips (*Scirtothrips dorsalis* (Hood)), *Thrips setosus* (Moulton), onion thrips *(T. tabaci* (Lindeman)), *F. intonsa* and *T. palmi*. Virus is acquired by the thrips only during their larval stages. Larvae can transmit the virus before they pupate but adults more commonly transmit the virus. Adults can remain infective throughout their life span.

The current distribution of TSWV covering all the continents makes it one of the most widely distributed plant viruses. The virus is widespread in temperate, subtropical and tropical climate zones throughout the world. At least 370 plant species representing 50 plant families, both mono- and dicotyledons, are naturally infected. The TSWV seriously affects the production of food and ornamental crops. Infections of plants with TSWV strains result generally in e.g. stunting, ringspots, dark purple-brown sunken spots, stem browning, flower breaking, necrotic and pigmental lesions and patterns, yellows and non-necrotic mottle, mosaic in greens or even total plant death. Most hosts only exhibit a part of these symptoms. The wide range of symptoms produced by TSWV has complicated the diagnosis and led to individual diseases being given several different names. Also, TSWV symptoms within the same plant species may vary depending on the age of the plant, time of infection during the life-cycle of the plant, nutritional levels and environmental conditions, especially temperature.

Although TSWV has been known for many years, is widely distributed, and causes economically important diseases in crops and ornamentals, limited progress has been made to identify sources for TSWV resistance genes. A multigenic TSWV tolerance has been identified in *Lycopersicon peruvianum*, but this resistance has not been transferred yet to cultivated tomatoes nor has a resistance source been identified for other crop species. The use of natural cross-protection systems to decrease the damage by severe TSWV strains is not well documented. Limited positive results have been reported for tomato and lettuce.

Therefore, the introduction of genetic information conferring resistance to tospovirus infection into plant gene pools by means of genetic manipulation provides breeder and grower a new method to combat tospoviral diseases.

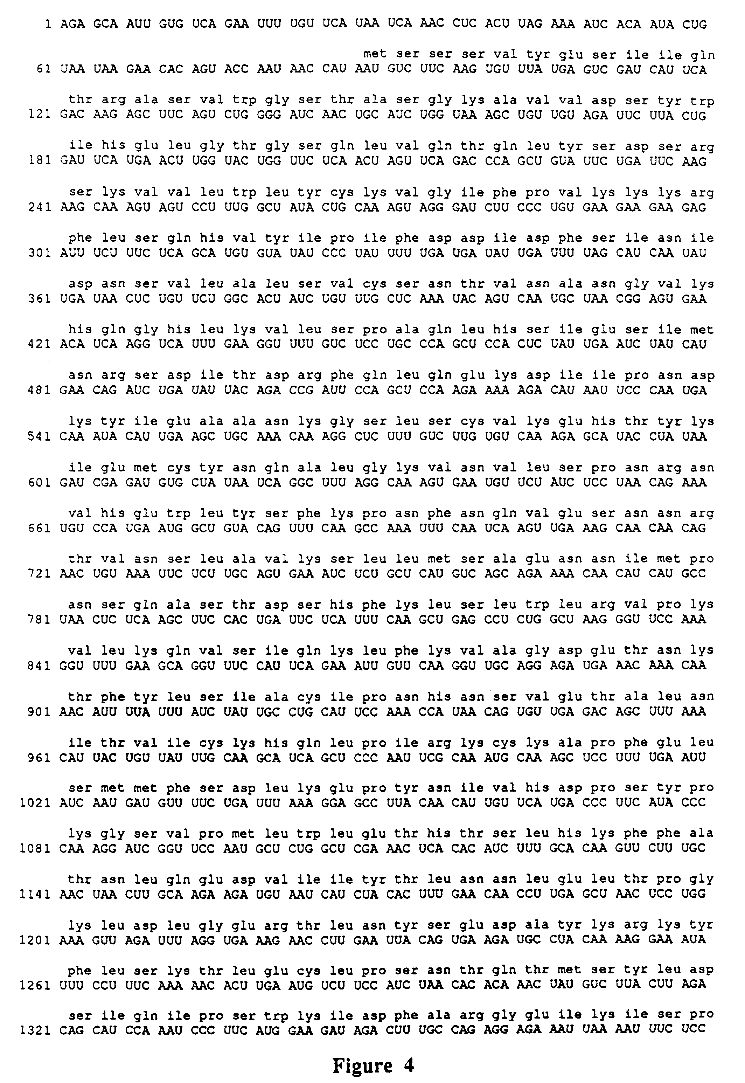

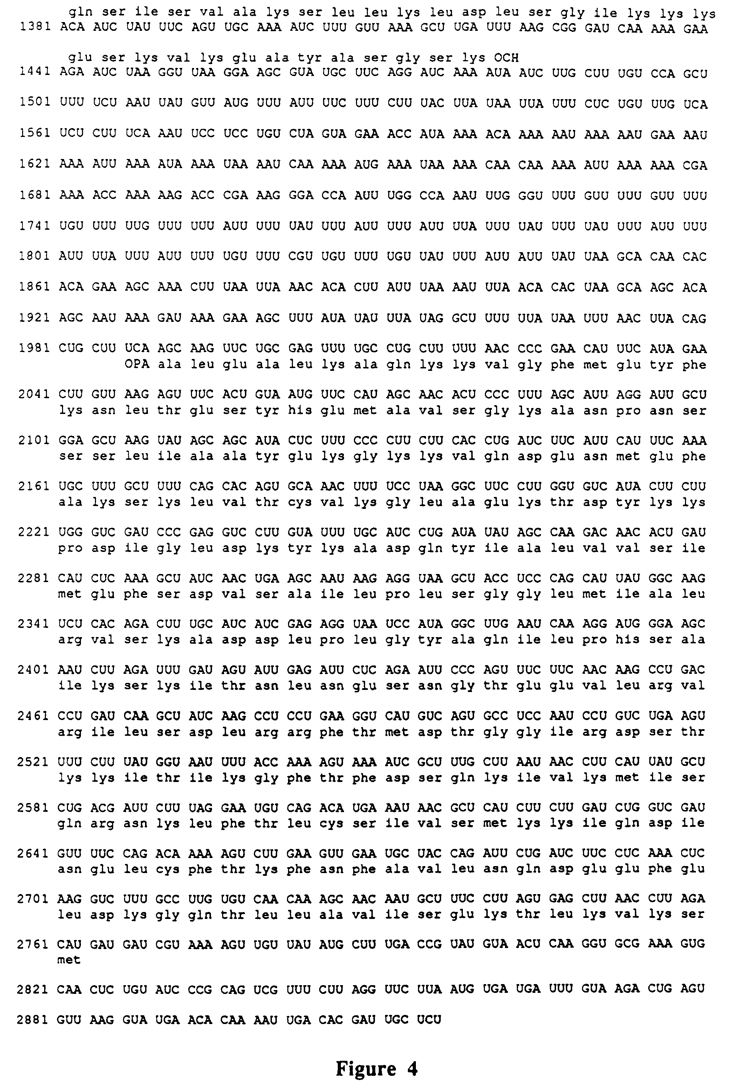

The present invention provides recombinant DNA constructs comprising a DNA sequence under expression control of a promoter and a terminator functioning in plants, which DNA sequence encodes:
(a) an RNA sequence selected from the group consisting of the following tospoviral sequences:
   (i) the S RNA nucleotide sequence from 1 to 2915 depicted in Figure 4;
   (ii) the S RNA nucleotide sequence from 2763 to 1987 depicted in Figure 4; or-
(b) an RNA sequence according to (a) encoding for a tospovirus protein in which one or more codons have been replaced by their synonyms (i.e. codons corresponding to the same amino acid or termination signal), or part thereof comprising at least 20 nucleotides, or an RNA sequence homologous thereto wherein a number of nucleotides have been deleted and/or added but which is still capable of hybridization to a nucleotide sequence complementary to an RNA sequence according to (a) under appropriate hybridization conditions; or -
(c) an RNA sequence complementary to an RNA sequence according to (a) or (b).

The DNA sequences defined under a), b) and c) hereinabove, are, hereinafter, for convenience referred to as "TSWV Related DNA Sequences". A TSWV Related DNA Sequence according to the invention may be modified, if desired, to create mutants or modified sequences homologous to a TSWV Related DNA Sequence from which they are derived using methods known to those skilled in the art such as site-directed mutagenesis. Such mutants or modified coding sequences are therefore within the spirit and the scope of this invention.

The term RNA sequence of a tospovirus refers to a sequence of the S RNA strand of a tospovirus as defined above.

The term RNA sequence homologous to an RNA sequence of a tospovirus refers to an RNA sequence of a tospovirus wherein a number of nucleotides have been deleted and/or added but which is still capable of hybridization to a nucleotide sequence complementary to an RNA sequence of a tospovirus under appropriate hybridization conditions. For the purpose of the invention appropriate hybridization conditions conveniently include an incubation for 16 hours at 42°C, in a buffer system comprising 5 x standard saline citrate (SSC), 0.5% sodium dodecylsulphate (SDS), 5 x Denhardt's solution, 50% formamide and 100 µg/ml carrier DNA (hereinafter the buffer system), followed by washing 3 times with a buffer comprising 1 x SSC and 0.1% SDS at 65°C for approximately one hour each time.

Preferred hybridization conditions for the purpose of the invention involve incubation in the buffer system for 16 hours at 49°C and washing 3 times with a buffer comprising 0.1 x SSC and 0.1% SDS at 55°C for approximately one hour each time. Most preferred hybridization conditions for the purpose of the invention involve incubation in the buffer system for 16 hours at 55°C and washing 3 times with a buffer comprising 0.1 x SSC and 0.1% SDS at 65°C for approximately one hour each time.

The length of the TSWV Related DNA Sequence will i.a. depend on the particular strategy to be followed, as will become apparent from the description hereinafter. In general, it will be desirable that the TSWV Related DNA Sequence comprises at least 20, suitably 50 or more nucleotides.

The term promoter as used herein refers to the nucleotide sequence upstream from the transcriptional start site and containing all the regulatory regions required for transcription including the region coding for the leader sequence of mRNA (which leader sequence comprises the ribosomal binding site and initiates translation at the AUG start codon).

Examples of promoters suitable for use in DNA constructs of the invention include viral, fungal, bacterial, animal and plant derived promoters functioning in plant cells. The promoter may express the DNA constitutively or differentially. Suitable examples of promoters differentially regulating DNA expression are promoters inducible by disease vectors, such as thrips, e.g. so-called wound inducible promoters. It will be appreciated that the promoter employed should give rise to the expression of a TSWV Related DNA Sequence at a rate sufficient to produce the amount of RNA necessary to decrease the tospovirus susceptibility of the transformed plant. The necessary amount of RNA to be transcribed may vary with the type of plant involved. Particularly preferred promoters include the cauliflower mosaic virus 35S (CaMV 35S) promoter, derivatives thereof, and a promoter inducible after wounding by a disease vector such as thrips, e.g. a wound inducible promoter.

The term terminator as used herein refers to a DNA sequence at the end of a transcriptional unit that signals termination of transcription. Terminators are DNA 3'-non-translated sequences that contain a polyadenylation signal, that causes the addition of polyadenylate sequences to the 3'-end of the primary transcript. Terminators active in plant cells are known and described in the literature. They may be isolated from for example bacteria, fungi, viruses, animals and plants. Examples of terminators particularly suitable for use in the DNA constructs of the invention include the nopaline synthase terminator of *A*. *tumefaciens*, the 35S terminator of CaMV and the zein terminator from *Zea mays*.

According to the terminology employed in the present specification, an RNA sequence is complementary to another RNA sequence if it is able to form a hydrogen-bonded complex with it, according to rules of base pairing under appropriate hybridization conditions (as defined hereinabove).

The invention also provides a vector capable of introducing the DNA construct of the invention into plants and methods of producing such vectors.

The term vector employed herein refers to a vehicle by means of which DNA fragments can be incorporated in a host organism.

The term plants is used herein in a wide sense and refers to differentiated plants as well as undifferentiated plant material such as protoplasts, plant cells, seeds, plantlets etc. that under appropriate conditions can develop into mature plants, the progeny thereof and parts thereof such as cuttings and fruits of such plants.

The invention further provides plants comprising in their genome a DNA construct of the invention, and methods of producing such plants.

The plants according to the invention have reduced susceptibility to diseases induced by tospoviruses and do not have the disadvantages and limitations of plants obtained by the classical methods as discussed hereinabove.

Examples of plants susceptible to tospoviruses such as TSWV include but are not limited to Ageratum, alfalfa, Amaranthus, Anthirrhinum, Aquilegia, aubergine, beet, Begonia, broad bean, broccoli, brussels sprouts, cabbage, cauliflower, celery, chicory, Chrysanthemum, Cineraria, clover, Cosmos, cowpea, cucumber, cyclamen, Dahlia, Datura, Delphinium, endive, Gerbera, Gladiolus, Gloxinia, gourd, groundnut, Hippeastrum, Impatiens, lettuce, melon, Mesembryanthemum, onion, papaya, pea, peanut, pepper, petunia, pineapple, potato, Primula, Saint Paulia, safflower, Salpiglossis, snap bean, soybean, spinach, squash, sugarbeet, sunflower, Tagetes, tobacco, tomato, Verbena, Vinca, watermelon, Zinnia. The invention relates in particular to these listed plants comprising in their plant genome a DNA construct of the invention.

Since TSWV is the typemember of the tospoviruses, the particular features of tospoviruses are hereinafter illustrated employing TSWV as an example.

The S, M and L RNA are single stranded RNA molecules. The S RNA is approximately 2,900 nucleotides long and comprises two genes, one encoding a non-structural protein (NSs) in viral sense, the other one encoding the nucleocapsid protein (N) in viral complementary sense. The intergenic region between the NSs- and N-gene can be folded into a hairpin structure. The 5'- and 3'-terminal sequences of the S RNA are capable of hybridizing to each other such that the first nucleotide is opposite (and complementary) to the last nucleotide of said S RNA strand. We designate hereinafter the double-stranded structure obtained by hybridizing both RNA termini for convenience "pan-handle".

The M RNA strand has approximately 5000 nucleotides. It contains one long open reading frame in viral complementary sense. This open reading frame is translated on polysomes located on the endoplasmic reticulum where the nascent polypeptide chain is cleaved co-translationally to form the spike proteins G₁ and G₂ respectively. Similar to the S RNA the termini of the M RNA strand are complementary to each other and may likewise hybridize to form a "pan-handle".

The L RNA strand has approximately 7500 nucleotides and contains one open reading frame in the viral complementary sense. This open reading frame most probably corresponds with the gene encoding the viral transcriptase with an estimated molecular weight of approximately 260 kd. In some mutant strains shortened L RNA molecules have been found in addition to the wild type, full-length L RNA. These shortened L RNAs however, do always possess the characteristic terminal nucleotide sequences and thus are capable of forming "pan-handle" structures. They are also encapsidated with nucleocapsid protein included in virus particles. Their presence supresses symptom development resulting in less severe detrimental effects. Hence, these shortened L RNA molecules can be regarded as defective interfering (DI) RNAs, a term known to those skilled in the art.

Another preferred embodiment of the invention relates to DNA constructs of the invention coding for transcription into tospovirus-RNA sequences of an open reading frame in viral complementary sense (i.e. having negative polarity), or into corresponding RNA sequences in which one or more codons have been replaced by their synonyms, or into RNA sequences homologous thereto.

Preferably the tospovirus-RNA sequences referred to hereinabove have at least 20, more preferably at least 50 nucleotides.

Examples of DNA constructs suitable for use according to the invention include TSWV Related DNA Sequences coding for transcription into (reference is made to Fig. 4 for S RNA nucleotide sequences):
i) the S RNA nucleotide sequence 1 to 2915;
ii) the S RNA nucleotide sequence 89 to 1483;
iii) the S RNA hairpin;
iv) the S RNA "pan-handle";
v) the S RNA nucleotide sequence 2763 to 1987;
vi) an RNA sequence complementary to the S RNA nucleotide sequence 1987 to 2763;
vii) an RNA sequence complementary to the S RNA nucleotide sequence 89 to 1483;
viii) S RNA nucleotide sequence 89 to 1483 in which one or more codons have been replaced by their synonyms;
ix) S RNA nucleotide sequence 2763 to 1987 in which one or more codons have been replaced by their synonyms.
x) an RNA sequence homologous to the nucleotide sequences defined under i) to vii) hereinabove;
xi) fragments of sequences defined under i) to x) hereinabove.

The DNA constructs of the invention may be obtained by insertion of the TSWV Related DNA Sequence in an appropriate expression vector, such that it is brought under expression control of a promoter functioning in plants and its transcription terminated by a terminator.

The term appropriate expression vector as used herein refers to a vector containing a promoter and a terminator which function in plant cells.

The insertion of the TSWV Related DNA Sequence into an appropriate expression vector may be carried out in a manner known per se. Suitable procedures are also illustrated by the examples hereinafter.

Likewise the construction of appropriate expression vector may be carried out in a manner known per se.

The plants according to the invention may be obtained by
a) inserting into the genome of a plant cell a DNA construct according to the invention;
b) obtaining transformed cells; and
c) regenerating from the transformed cells genetically transformed plants.

The DNA vectors of the invention may be inserted into the plant genome of plants susceptible to TSWV infection. Such plant transformation may be carried out employing techniques known per se for the transformation of plants, such as the plant transformation techniques involving the Ti plasmids derived from *Agrobacterium tumefaciens, A*. *rhizogenes* or modifications thereof, naked DNA transformation or electroporation of isolated plant cells or organized plant structures, the use of micro-projectiles to deliver DNA, the use of laser systems, liposomes, or viruses or pollen as transformation vectors and the like.

The plants of the invention may be monitored for expression of a TSWV Related DNA Sequence by methods known in the art, including Northern analysis, Southern analysis, PCR techniques and/or immunological techniques. The plants of the invention show decreased susceptibility to TSWV infection as demonstrated by tests whereby said plants are exposed to TSWV preferentially at a concentration in the range where the rate of disease symptoms correlates linearly with the TSWV concentration in the inoculum.

Methods suitable for TSWV inoculation are known in the art; they include mechanical inoculation and, in particular, the use of appropriate vectors.

The plants of the invention may of course also be obtained by crossing of a obtained plant according to the methods of the invention with another plant to produce plants having in their plant genome a DNA construct of the invention.

The invention is illustrated by the following non-limitative examples and the attached figures.
Figure 1 gives an overview of the structure of the tomato spotted wilt virus.
Figure 2 gives a review of the cloning strategy employed for TSWV S RNA.
Figure 3 gives a review of the cloning strategy employed for TSWV L RNA.
Figure 4 shows the sequence and genomic organization of the TSWV S RNA. The amino acid sequence of the non-structural protein NSs (89-1483) is outlined above, that of the nucleocapsid protein N (2763-1987) under the corresponding RNA nucleotide sequence.
Figure 5 gives the distribution of translation initiation and termination codons for all reading frames of the S RNA (fig. 5A) and the L RNA (fig. 5B). Full bars indicate stop codons, half bars ATG start codons.
Figure 6 shows the nucleotide sequence of the TSWV M RNA (fig. 6A) and the L RNA (fig. 6B) as elucidated uptill now.
Figure 7 gives a schematic review of the construction of a suitable expression vector (pZU-A).
Figure 8 gives a schematic review of the construction of a suitable expression vector (pZU-B).
Figure 9 gives a schematic review of the construction of a suitable plasmid (pTSWV-N) comprising the nucleocapsid N protein gene.
Figure 10 gives a schematic review of the construction of a suitable plasmid (pTSWV-NSs) comprising the nucleocapsid NSs-protein gene.
Figure 11 gives a schematic example of the construction of a plant transformation vector pTSWV-NAB, a DNA construct according to the invention.
Figure 12 gives a schematic example of the construction of a plant transformation vector pTSWV-Nmut BB, a DNA construct according to the invention.
Figure 13 gives a schematic example of the construction of a plant transformation vector pTSWV-NSsAB, a DNA construct according to the invention.
Figure 14 gives a schematic example of the construction of a plant transformation vector pTSWV-NSsmut BB, a DNA construct according to the invention.
Figure 15 shows the "pan-handle" region of TSWV S RNA.
Figure 16 shows the hairpin region of TSWV S RNA.
Figure 17 shows a dot blot analysis of suspected plants.

Suitable examples of preferred TSWV Related DNA Sequences coding for transcription into a sequence of the hairpin structure of S RNA or of RNA sequences homologous thereto are sequences coding for the 1583-1708 nucleotide sequence of S RNA, for the 1709-1835 nucleotide sequence of S RNA, for the 1583-1835 nucleotide sequence of the S RNA or for a sequence homologous to such sequences.

Suitable examples of preferred TSWV Related DNA Sequences coding for transcription into a sequence of the "pan-handle" region of S RNA or of RNA sequences homologous thereto is the combination of the sequences coding for the 1-70 and 2,850-2916 nucleotide sequence of S RNA, or for sequences homologous to such sequences.

Suitable examples of preferred TSWV Related DNA Sequences coding for transcription into a "pan-handle" region of L RNA or of RNA sequences homologous thereto is the combination of sequences coding for the first 80, in particular the first 65 nucleotides from the 5'-end of the viral L RNA and the last 80, in particular the last 65 nucleotides from the 3'-end of the viral L RNA, or for sequences homologous to such sequences or derivatives thereof.

Other objects, features, advantages of the present invention will become apparent from the following examples.

References are abbreviated to the first authors name, full references are listed later.

### Material and methods

All TSWV RNA derived sequences presented in here are depicted as DNA sequences for the sole purpose of uniformity only. A person skilled in the art appreciates that this is done for convenience only.

Cultivars of *Nicotiana tabacum* and *Petunia hybrida*, used in plant transformation studies, are grown under standard greenhouse conditions. Axenic explant material is grown on standard MS media (Murashige and Skoog, 1962) containing appropriate phytohormones and sucrose concentrations.

*E. coli* bacteria are grown on rotary shakers at 37°C in standard LB-medium. *Agrobacterium tumefaciens* strains are grown at 28°C in MinA medium supplemented with 0.1 % glucose (Ausubel *et al*., 1987).

In all cloning procedures the *E*. *coli* strain JM83, (F⁻, *Δ*(*lac-pro*), *ara*, *rps*L, ⌀80, d*lac*ZM15) is used as a recipient for recombinant plasmids.

Binary vectors are conjugated to *Agrobacterium tumefaciens* strain LBA 4404, a strain containing the Ti-plasmid *vir* region, (Hoekema *et al*., 1983) in standard triparental matings using the *E*. *coli* HB101, containing the plasmid pRK2013 as a helper strain. (Figurski and Helinski, 1979). Appropriate *Agrobacterium tumefaciens* recipients are selected on media containing rifampicin (50 µg/ml) and kanamycine (50 µg/ml).

Cloning of fragments in the vectors pUC19 (Yanish-Perron *et al*., 1985), pBluescript (Stratagene), pBIN19 (Bevan *et al*., 1984) or derivatives, restriction enzyme analysis of DNA, transformation to *E*. *coli* recipient strains, isolation of plasmid DNA on small as well as large scale, nick-translation, *in vitro* transcription, DNA sequencing, Southern blotting and DNA gel electrophoresis are performed according to standard procedures (Maniatis *et al*., 1982; Ausubel et al., 1987).

### Examples

### Example 1: Isolation of TSWV particles and the genetic material therein

Tomato spotted wilt virus strain CPNH1, a Brazilian isolate from tomato, is maintained on tomato by grafting. Virus is purified from systemically infected *Nicotiana rustica* leaves, after mechanical inoculation essentially as described by Tas *et al*. (1977). It is essential to maintain all material used in this isolation procedure at 4 °C. Twelve days after inoculation of 100 grams of infected leaves are harvested and ground 5 - 10 seconds at low speed setting in 5 volumes extraction buffer (0.1 M NaH₂PO₄, 0.01 M Na₂SO₃, pH 7) in a Waring blender. The suspension is filtered through cheesecloth and the filtrate is centrifuged for 10 minutes at 16,000 x *g*. The resulting pellet is resuspended in three volumes resuspensionbuffer (0.01 M NaH₂PO₄, 0.01 M Na₂SO₃, pH 7). The pellet is dissolved by stirring carefully at 4 °C. After centrifugation for 10 minutes at 12,500 x *g* the pellet is discarded and the supernatant is centrifuged again for 20 minutes at 50,000 x *g*. The pellet is resuspended in 0.2 volume of resuspensionbuffer (0.01 M NaH₂PO₄, 0.01 M Na₂SO₃, pH 7) and kept on ice for 30 minutes. Antiserum raised in rabbits against material from non-infected *Nicotiana rustica* is added to the solution, carefully stirred for 1 hour and non-viral complexes are pelleted by 10 minutes centrifugation at 16,000 x *g*. The cleared supernatant is loaded on a linear 5 - 40 % sucrose gradient in resuspensionbuffer: 0.01 M NaH₂PO₄, 0.01 M Na₂SO₃, pH 7, and spun for 45 minutes at 95,000 x *g*. The opalescent band containing TSWV virions is carefully collected with a syringe and diluted 4 times with resuspensionbuffer. Washed virions are pelleted by centrifugation for 1.5 hours at 21,000 x *g* and resuspended in one volume resuspensionbuffer. Generally, 100 grams of leaf material yields approximately 0,5 mg of TSWV virions. TSWV RNA is recovered preferentially from purified virus preparations by SDS-phenol extractions followed by ethanol precipitation. From 1 mg TSWV 1-5 µg of RNA is extracted. The intactness of the isolated RNA molecules is analysed by electrophoresis on an agarose gel. Three distinct RNA molecules are identified with apparent sizes of 2900 nucleotides (S RNA), 5000 nucleotides (M RNA) and 7500 nucleotides (L RNA) respectively.

### Example 2: Sequence determination of the 3'-termini of the TSWV viral RNAs

For direct RNA sequencing TSWV RNA is extracted from purified nucleocapsids essentially according to Verkleij *et al*. (1983). Twelve days after inoculation 100 grams of infected leaves are harvested and ground 5 - 10 seconds at low speed setting in four volumes TAS-E buffer (0.01 M EDTA, 0.01 M Na₂SO₃, 0.1 % cysteine, 0.1 M TRIS pH 8.0) in a Waring blender. The suspension is filtered through cheesecloth and centrifuged 10 minutes at 1,100 x *g*. Nucleocapsids are recovered from this supernatant by 30 minutes centrifugation at 66,000 x *g*. The pellet is carefully resuspended in one volume TAS-R buffer (1 % Nonidet NP-40, 0.01 M EDTA, 0.01 M Na₂SO₃, 0.1 % cysteine, 0.01 M glycine, 0.01 M TRIS pH 7.9). The pellet is dissolved by stirring carefully for 30 minutes at 4 °C. The supernatant is cleared by a 10 minutes centrifugation at 16,000 x *g*. Crude nucleocapsids are collected from the cleared supernatant by sedimentation through a 30 % sucrose cushion for 1 hour at 105,000 x *g*. The nucleocapsid pellet is resuspended in 400 µl 0.01 M Na-citrate pH 6.5, layered on a 20 - 40 % sucrose (in 0.01 M Na-citrate pH 6.5) and spun for 2 hours at 280,000 x *g*. The three different opalescent bands, respectively L, M and S nucleocapsid, are collected separately. TSWV RNA is recovered preferentially from purified nucleocapsid preparations by SDS-phenol extractions followed by ethanol precipitation. Routinely 100 µg of RNA are obtained from 100 grams of infected leaves. The 3'-ends of the separate TSWV RNAs are labeled using RNA ligase and 5'-[³²P]pCp. These end-labeled RNA molecules are separated on a low gelling temperature agarose gel (Wieslander, 1979). The enzymatic approach described by Clerx-Van Haaster and Bishop (1980) and Clerx-Van Haaster *et al*. (1982) is used to determine the 30 terminal nucleotides of the 3'- and 5'-ends of both S and M RNA.

Synthetic oligonucleotides complementary to the 3'-termini are synthesized using a commercially available system (Applied Biosystems) and used for dideoxy-sequencing with reverse transcriptase.

### Example 3: cDNA cloning of TSWV genetic material

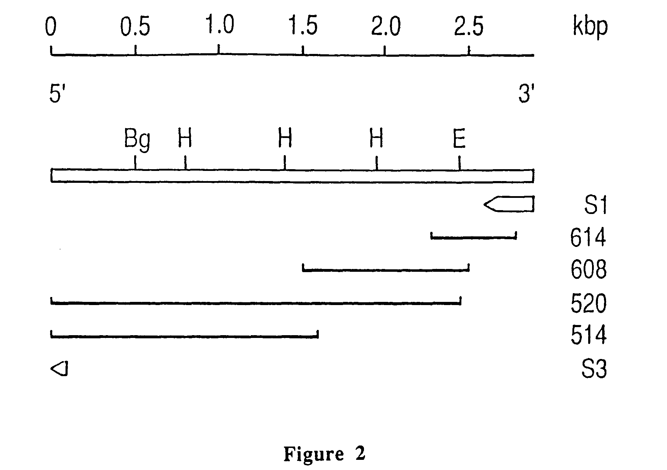

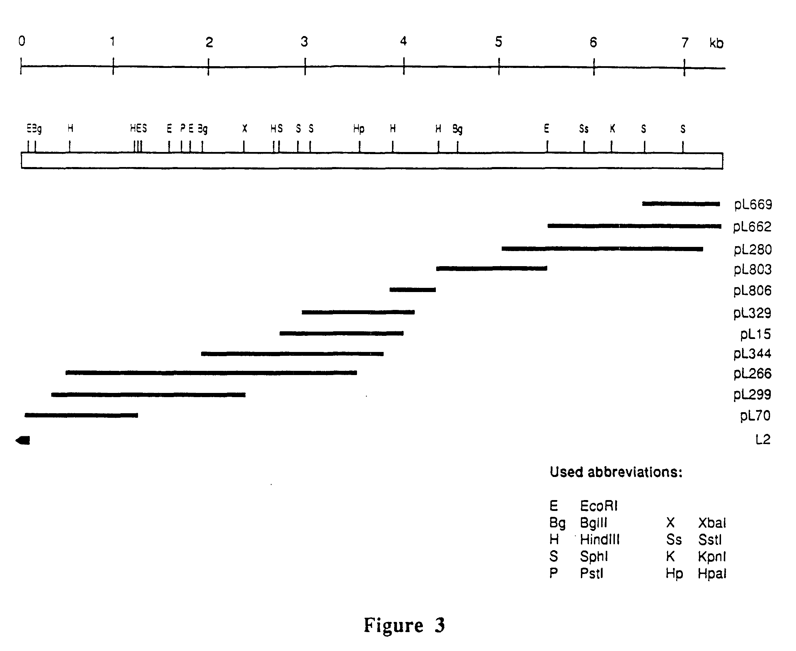

Oligonucleotides complementary to the 3'-ends of the S and L RNA are used for priming first strand cDNA synthesis. With these primers, double stranded DNA to TSWV RNA is synthesized in principle according to Gubler and Hoffman (1983). Two different approaches are used to generate cDNA clones to the TSWV viral RNAs. A first series of clones is obtained by random priming of the TSWV RNA using fragmented single stranded calf thymus DNA, followed by first and second strand cDNA synthesis. cDNA is made blunt-ended using T4-DNA polymerase and ligated with T4 ligase into the SmaI site of pUC19. A second series of TSWV cDNA clones is obtained by priming first strand DNA synthesis with the oligonucleotides complementary to the 20 terminal nucleotides at the 3'-ends of the TSWV RNAs. After second strand synthesis, treatment with T4 DNA polymerase to create blunt ends, phosphorylated EcoRI linkers are ligated to the ends of the cDNAs essentially according to Huynh *et al*. (1985). After restriction of these cDNA molecules with EcoRI, the cDNA fragments are ligated in the EcoRI site of lambda gt10. cDNA clones from both series containing viral inserts are selected via colony hybridization, essentially according to Grunstein and Hogness (1975) using [³²]P-labeled, randomly primed first strand cDNA as a probe. Sets of overlapping cDNA clones are selected by Southern analysis followed by plasmid and/or phage walking, in order to construct restriction maps, based on cDNA derived sequences of the S (figure 2) and L RNA (figure 3).

### Example 4: Sequence determination of the TSWV S, M and L RNA

In order to determine the sequence of the S RNA 4 selected cDNA clones are subcloned in M13mp18, resulting in the plasmids pS614, pS608, pS520 and pS514, as described above (fig. 2). These clones are sequenced in both directions using the standard protocol of Yanish-Perron *et al*. (1985). The nucleotide sequence of the 3'-end of the S RNA is determined by primer extension of the synthetic oligonucleotide S1 (5' d(GAGCAATCGTGTCAATTTTG)), which is complementary to the 20 nucleotides of the 3'-terminus. A second synthetic oligonucleotide S3 complementary to the nucleotides 30 to 50 from the 5'-end of the viral S RNA is used to verify the 5'-terminal sequence of the S RNA by primer extension. Sequence data from the TSWV SRNA (2916 nt) are summarized in figure 4.

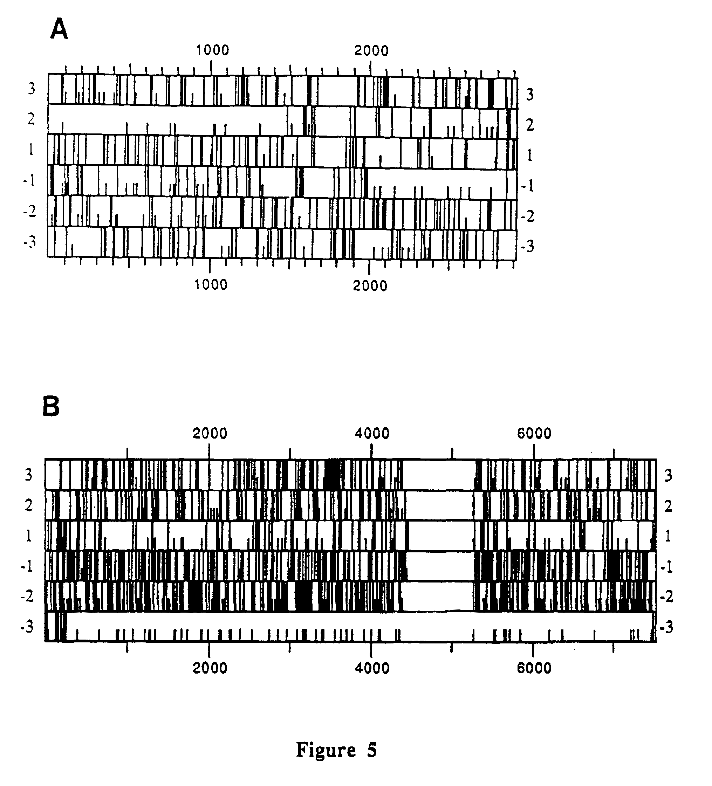

Computer simulated translation of the 6 different reading frames on the viral strand and viral complementary strand reveals the presence of two putative open reading frames (see figure 5A). On the viral strand an open reading frame is found starting at position 89 and terminating at an UAA stopcodon at position 1483 possibly encoding a protein of 464 amino acids with a molecular weight of 52.4 kd. This protein is most likely a non-structural protein, tentatively designated NSs. The other open reading frame is located on the viral complementary strand from position 2763 to 1987, encoding a 258 amino acids long polypeptide with a molecular weight of 28.8 kd. This open reading frame possibly encodes the viral nucleocapsid protein N.

To verify this assumption the amino acid composition of the purified nucleocapsid protein is determined and compared with the deduced composition from the sequence data. Also, the putative nucleocapsid encoding N protein gene is inserted into pBluescript. Using T7 RNA polymerase the N protein gene is transcribed *in vitro.* This *in vitro* synthesized transcript is subsequently translated in an *in vitro* rabbit reticulocyte lysate (New England Nuclear: NEN) system in the presence of [³⁵S] labeled methionine (NEN). A protein with a molecular weight identical to the native nucleocapsid protein could be precipitated from the synthesized radioactive proteins, when antibodies raised against purified nucleocapsid protein are used, following procedures essentially as described (Van Grinsven *et al*, 1986). This indicates that the N protein gene encodes the viral nucleocapsid protein. Figure 5A shows the coding capacities of the viral and the viral complementary strand of the S RNA indicating the NSs and N protein gene respectively, both genes being expressed from subgenomic mRNAs. Thus, the unique situation occurs that a plant virus RNA has an ambisense gene arrangement. Other important features of this S RNA sequence is the existence of complementary terminal repeats capable of forming so-called "pan-handle" structures. These structures play an important role in replication and transcription of the viral RNA. Another putative regulatory element is the hairpin stucture in the intergenic region of the S RNA, which most likely contains the transcription termination signals for both subgenomic mRNAs, encoding respectively the N and NSs-protein.

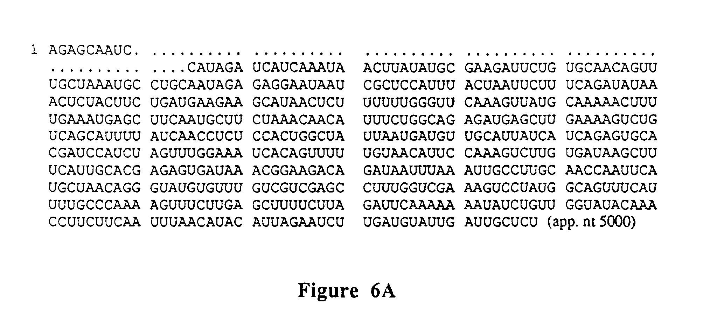

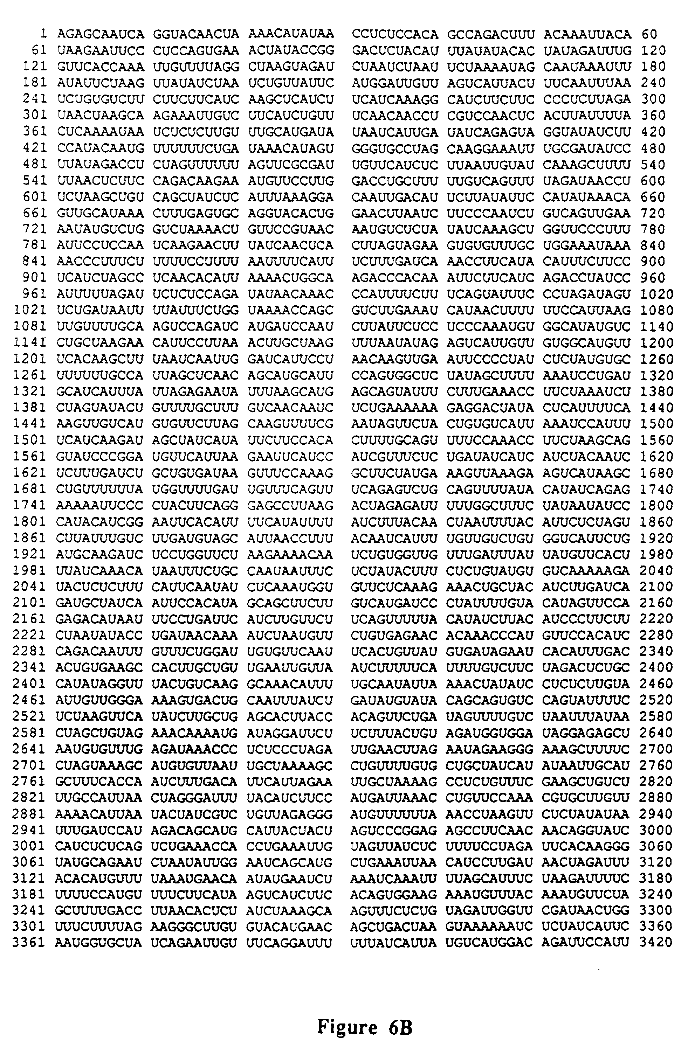

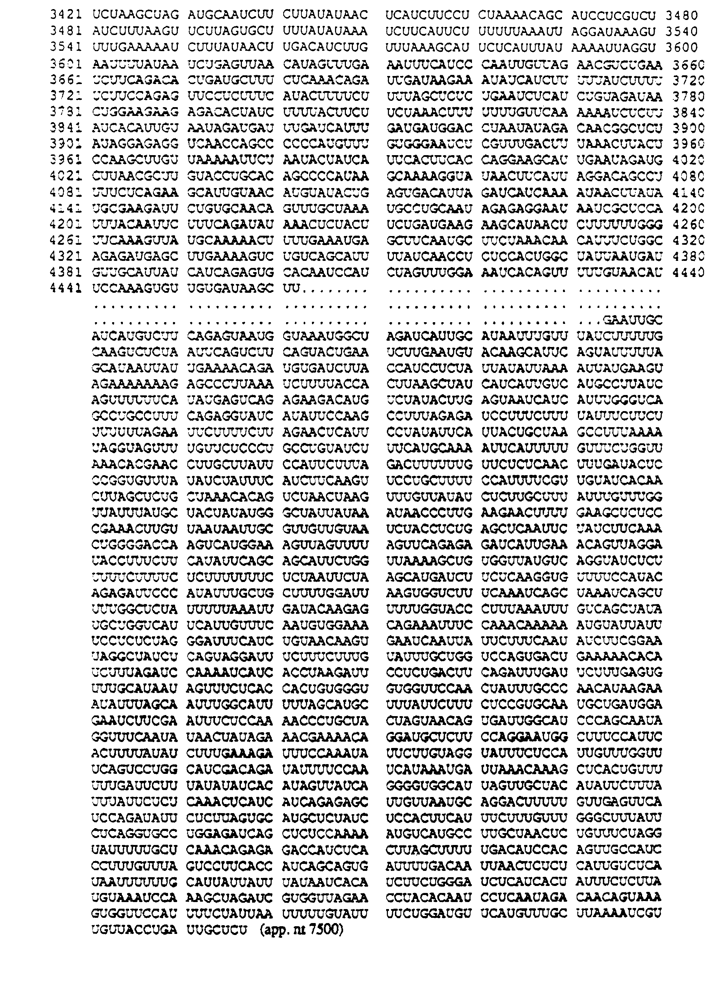

The nucleotide sequence of the TSWV M and L RNA was elucidated employing the same strategies and methods followed to determine the nucleotide sequence of the S RNA. Figure 3 shows the cloning strategy for the L RNA derived, overlapping cDNA clones. Summarized sequence data of the TSWV M and L RNA are given in figure 6A and 6B respectively. Computer simulated translation suggests that this viral L RNA is of negative polarity containing one single open reading frame which starts 34 nucleotides from the 3'-end of the viral RNA and stops at nucleotide 236 from the 5'-end of the viral RNA (figure 5B). The mRNA (complementary to the viral L RNA) containing this open reading frame is most probably translated into the viral transcriptase.

### Example 5: Construction of an expression vector pZU-A

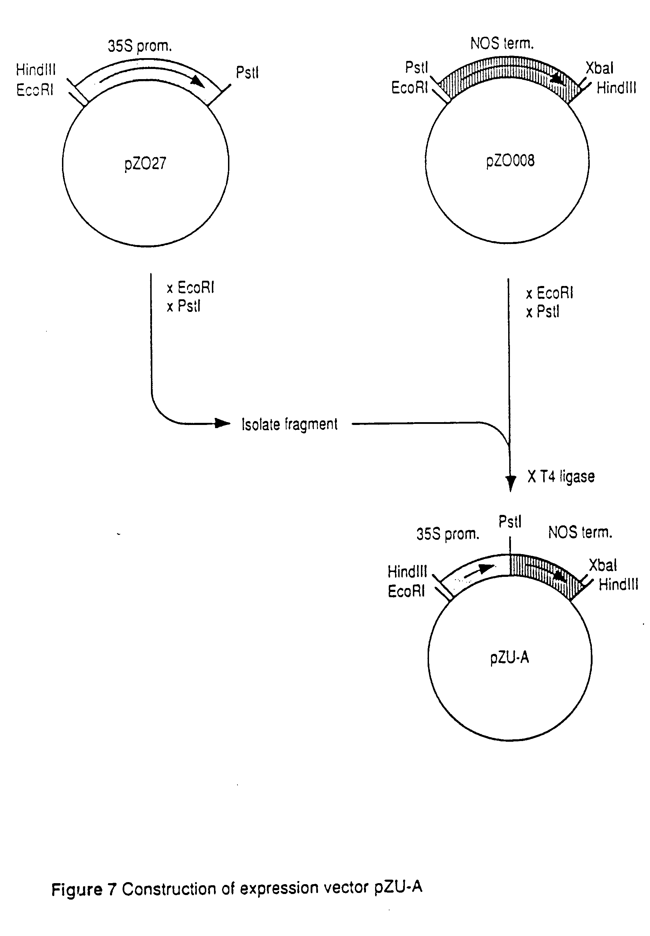

The 35S cauliflower mosaic virus (CaMV) promoter fragment is isolated from the recombinant plasmid pZO27, a derivative of pUC19 carrying as a 444 bp HindIII-PstI fragment the HincII-HphI region of the 35S promoter of CaMV strain Cabb-S (Franck et al., 1980). The nucleotide sequences of CaMV strains are very similar for the different strains. The 35S promoter fragment is excised from pZO27 as a 472 bp EcoRI-PstI fragment which contains: a part of the polylinker region, 437 bp of the non-transcribed region, the transcription initiation site and 7 bp of the non-translated leader region but not containing any 35S translational initiators. This 35S promoter fragment is ligated using T4 ligase into EcoRI-PstI linearized pZO008. This plasmid pZO008 carries the nopaline synthase (NOS) terminator as a 270 bp PstI-HindIII fragment. The resulting recombinant plasmid pZU-A carries the 35S promoter, a unique PstI site and the NOS terminator (figure 7). The sequence of the used 35S promoter in the plant expression vector pZU-A is as follows :

1 AAGCTTCTAG AGATCCGTCA ACATGGTGGA GCACGACACT CTCGTCTACT
51 CCAAGAATAT CAAAGATACA GTCTCAGAAG ACCAAAGGGC TATTGAGACT
101 TTTCAACAAA GGGTAATATC CGGAAACCTC CTCGGATTCC ATTGCCCAGC
151 TATCTGTCAC TTCATCAAAA GGACAGTAGA AAAGGAAGGT GGCACCTACA
201 AATGCCATCA TTGCGATAAA GGAAAGGCTA TCGTTCAAGA TGCCTCTGCC
251 GACAGTGGTC CCAAAGATGG ACCCCCACCC ACGAGGAGCA TCGTGGAAAA

301 AGAAGACGTT CCAACCACGT CTTCAAAGCA AGTGGATTGA TGTGATATCT
351 CCACTGACGT AAGGGATGAC GCACAATCCC ACTATCCTTC GCAAGACCCT
401 TCCTCTATAT AAGGAAGTTC ATTTCATTGG AGAGGACCCT GCAG

The complete sequence from the used NOS terminator in the vector pZU-A is as follows:

1 CTGCAGATCG TTCAAACATT TGGCAATAAA GTTTCTTAAG ATTGAATCCT
51 GTTGCCGGTC TTGCGATGAT TATCATATAA TTTCTGTTGA ATTACGTTAA
101 GCATGTAATA ATTAACATGT AATGCATGAC GTTATTTATG AGATGGGTTT
151 TTATGATTAG AGTCCCGCAA TTATACATTT AATACGCGAT AGAAAACAAA
201 ATATAGCGCG CAACCTAGGA TAAATTATCG CGCGCGGTGT CATCTATGTT
251 ACTAGATCTC TAGAAAGCTT

### Example 6: Construction of an expression vector pZU-B

The recombinant plasmid pZO347 is a derivative of pBluescript carrying a 496 bp BamHI-SmaI fragment containing a 426 bp 35S promoter fragment (HincII fragment) of CaMV strain Cabb-S, linked to a 67 bp fragment of the non-translated leader region, the so-called Ω-region, of the tobacco mosaic virus. This results in a chimeric promoter with a complete transcriptional fusion between the promoter of CaMV to the untranslated leader of TMV. By using *in vitro* mutagenesis the original position of the TMV ATG startcodon is mutated to a SmaI site.

The plasmid pZO008 carries the nopaline synthase (NOS) terminator as a 260 bp PstI-HindIII fragment. This PstI-HindIII fragment is excised from pZO008 and ligated using T4 ligase into PstI-HindIII linearized pZO347. The resulting recombinant plasmid pZU-B is another plant expression vector. The sequence of this 35S-Ω promoter as used in the plant expression vector pZU-B is as follows:

1 GGATCCGGAA CATGGTGGAG CACGACACGC TTGTCTACTC CAAAAATATC
51 AAAGATACAG TCTCAGAAGA CCAAAGGGCA ATTGAGACTT TTCAACAAAG
101 TTATTGTGAA GATAGTGGAA AAGGAAGGTG GCTCCTACAA ATGCCATCAT
151 TGCGATAAAG GAAAGGCCAT CGTTGAAGAT GCCTCTGCCG ACAGTGGTCC
201 CAAAGATGGA CCCCCACCCA CGAGGAGCAT CGTGGAAAAA GAAGACGTTC
251 CAACCACGTC TTCAAAGCAA GTGGATTGAT GTGATATCTC CACTGACGTA
301 AGGGATGACG CACAATCCCA CTATCCTTCG CAAGACCCTT CCTCTATATA

351 AGGAAGTTCA TTTCATTTGG AGAGGACTTT TTACAACAAT TACCAACAAC
401 AACAAACAAC AAACAACATT ACAATTACTA TTTACAATTA CCCGGG

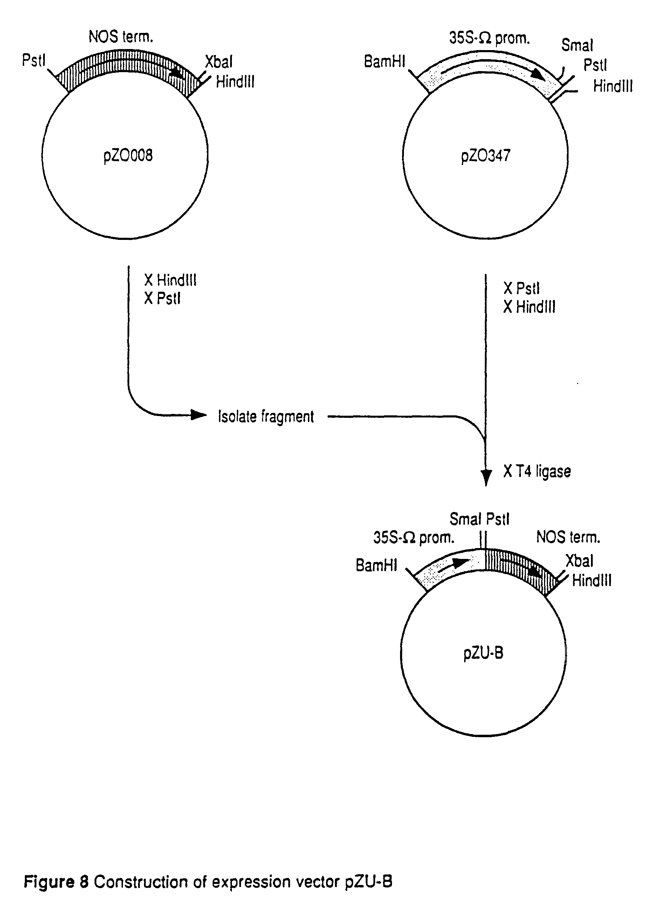

The resulting recombinant plasmid pZU-B contains the 35S HincII-TMV Ω fusion (35S-Ω), unique SmaI and PstI sites and the NOS terminator (figure 8). This expression vector is preferentially used in constructing translational fusions of the gene to be expressed downstream of the chimeric promoter 35S-Ω.

### Example 7: Subcloning of the TSWV-N protein gene

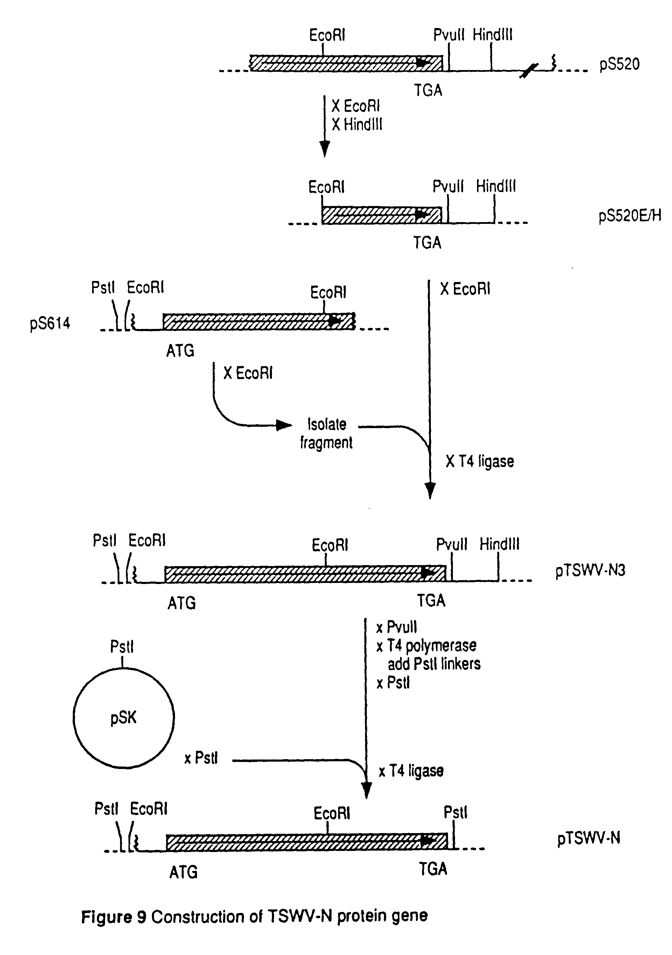

The TSWV-N protein coding sequence is obtained by fusion of the cDNA clones pS614 and pS520 (see figure 2). The cDNA clone pS520 is subjected to EcoRI-HindIII double-digestion and the fragment containing the 3'-end of the TSWV-N protein gene is separated electrophoretically and purified from the gel using a DEAE membrane (NA-45, Schleicher and Schüll) and cloned in the EcoRI-HindIII linearized pBluescript (Stratagene) resulting in the recombinant plasmid pS520E/H. The 5'-end containing fragment of the TSWV-N protein is excised from pS614 by a EcoRI digestion. This fragment is separated electrophoretically and purified from the gel using a DEAE membrane (NA-45, Schleicher and Schüll) and cloned in the EcoRI linearized pS520E/H resulting in the recombinant plasmid pTSWV-N3. The TSWV-N gene containing plasmid pTSWV-N3 is linearized by digestion with PvuII. PstI linkers 5' d(CCTGCAGG) are ligated with T4 ligase to the blunt ends of the linear DNA. Subsequently, the DNA is digested with PstI and the fragment containing the TSWV-N protein sequence is separated electrophoretically and excised from the gel. The TSWV-N protein gene containing fragment is ligated into a PstI linearized vector such as pBluescipt (Stratagene) to yield the recombinant plasmid pTSWV-N (figure 9). This addition of restriction sites facilitates the construction of further plasmids. (Alternatively, one may choose to add the sites in different ways such as but not limited to site-directed mutagenesis or by ligation of other synthetic oligonucleotide linkers. These methods are all known to a person skilled in the art.)

### Example 8: Subcloning of the TSWV non-structural protein gene (NSs-gene) of the TSWV S RNA

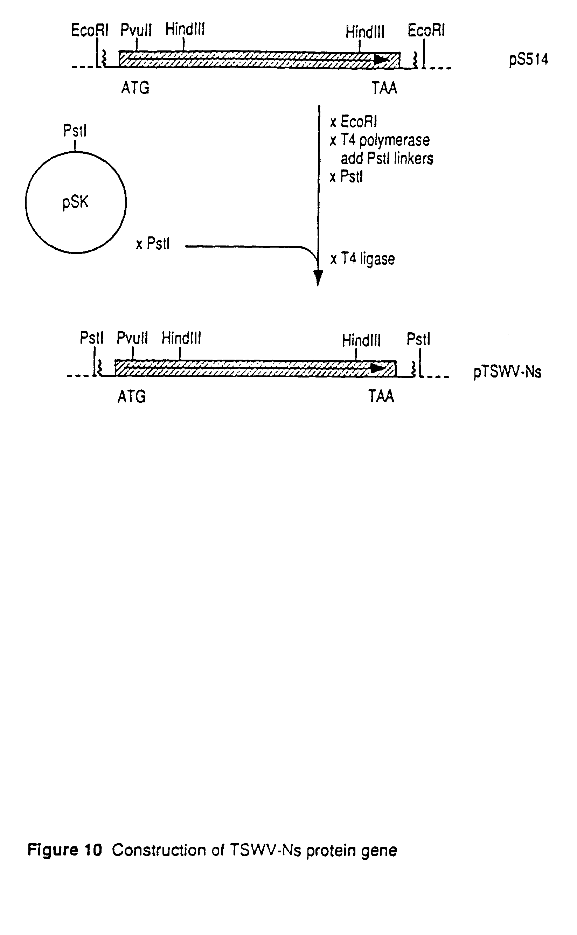

The sequence of the non-structural protein NSs is isolated from the cDNA clone pS514. The NSs-protein gene is located on an EcoRI fragment. After restriction of the cDNA clone pS514 with EcoRI and treatment with T4 DNA polymerase to create blunt ends. The NSs gene containing fragment is separated electrophoretically on an agarose gel and excised from the gel. To this blunt ended fragment containing the NSs-protein gene synthetic PstI linkers (5' d(CCTGCAGG)-3') are ligated using T4 polymerase. After restriction with PstI, the NSs-protein gene containing fragment is ligated in an PstI linearized pBluescript to yield the recombinant plasmid pTSWV-NSs (figure 10).

### Example 9: Construction of plant transformation vectors containing TSWV sequences

### Example 9A: N-protein gene constructions in pZU-A

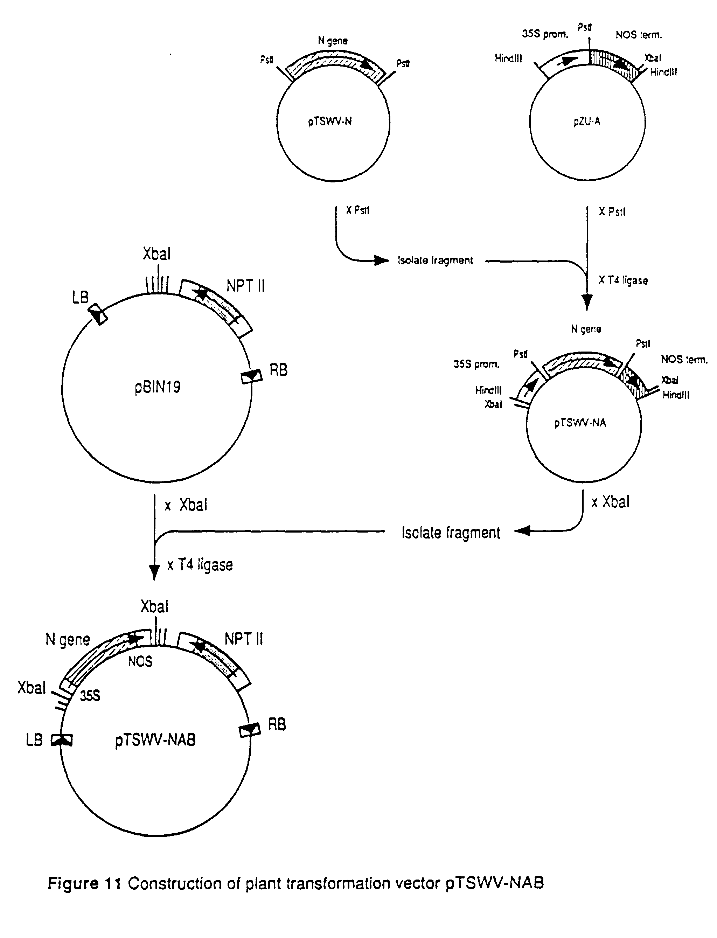

In order to create a plant transformation vector containing the N protein gene driven by the 35S promoter and terminated by the NOS terminator, the PstI fragment of pTSWV-N is isolated and inserted into PstI linearized pZU-A thereby creating the chimeric gene cassette vector pTSWV-NA. The cassette containing the 35S promoter, the N-protein gene and the NOS terminator is excised from pTSWV-NA by restriction with XbaI and ligated in the unique XbaI site of pBIN19, a binary transformation vector developed by Bevan *et al* (1984). The resulting plasmid pTSWV-NAB (figure 11) is used in plant transformation experiments using methods well known to a person skilled in the art.

### Example 9B: N-protein constructions in pZU-B

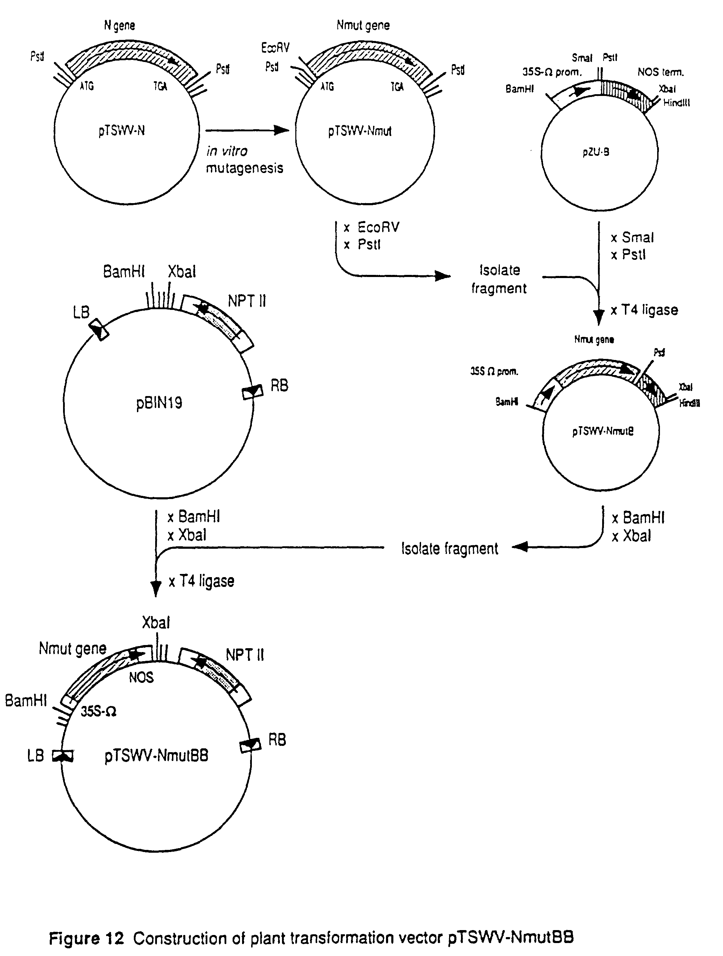

In order to make a fusion in which the ATG start codon from the N protein gene is fused directly to the 3'-end of the TMV untranslated leader of the 35S-Ω promoter the startcodon of the N gene has to be mutated. Using site-directed mutagenesis, the sequence 5' d(ACGATCATC**ATG**TCT) in pTSWV-N is mutated to 5' d(ACGATATC**ATG**TCT), thereby creating an EcoRV site: 5' d(GAT|ATC) just proximal to the ATG startcodon of the N gene. The resulting recombinant plasmid is called pTSWV-Nmut. The mutated N protein gene is excised from this plasmid via an EcoRV-PstI digestion. This fragment is isolated and inserted into the SmaI-PstI linearized pZU-B, resulting in recombinant plasmid pTSWV-NmutB. The chimeric cassette containing the 35S-Ω promoter, the mutated N gene and the NOS terminator is excised from the plasmid pTSWV-NmutB via a BamHI/XbaI digestion. The isolated chimeric gene cassette is then inserted into the BamHI/XbaI linearized pBIN19 to create the binary transformation vector pTSWV-NmutBB. The resulting plasmid pTSWV-NmutBB (figure 12) is used in plant transformation experiments using methods well known to a person skilled in the art

### Example 9C: NSs-protein gene constructions in pZU-A

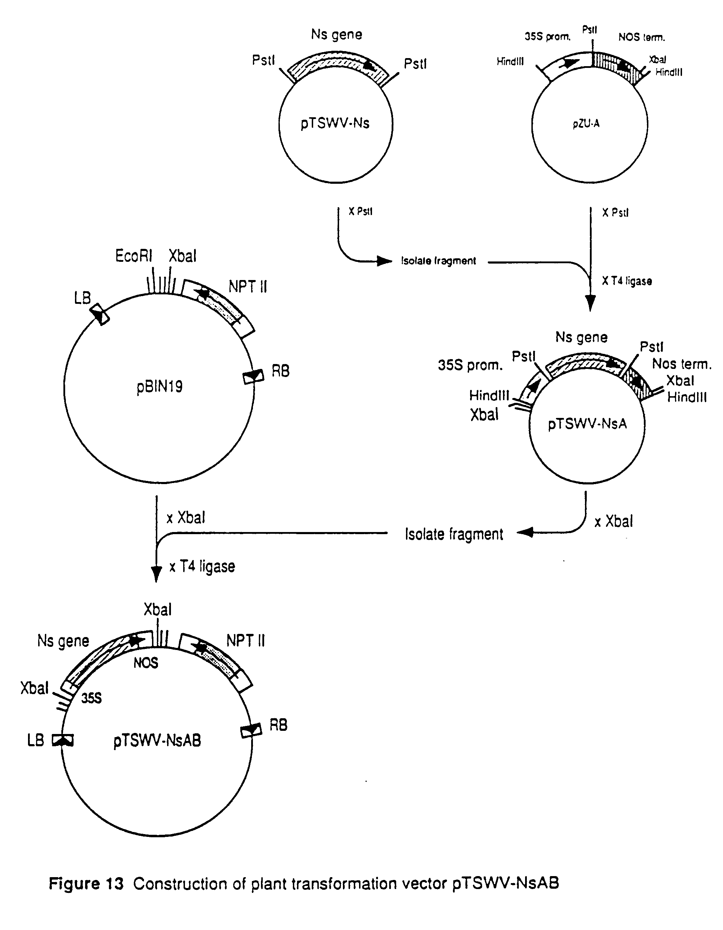

In order to create a plant transformation vector containing the NSs-protein gene driven by the 35S promoter and terminated by the NOS terminator, the PstI fragment of pTSWV-NSs is isolated and inserted into PstI linearized pZU-B to create the chimeric gene cassette vector pTSWV-NsA. The cassette containing the 35S promoter, the NSs-protein gene and the NOS terminator is excised from pTSWV-NsA by restriction with EcoRI and XbaI and ligated into EcoRI-XbaI linearized pBIN19. The resulting plasmid pTSWV-NsAB (figure 13) is used in plant transformation experiments using methods well known to a person skilled in the art.

### Example 9 D: NSs-protein gene constructions in pZU-B

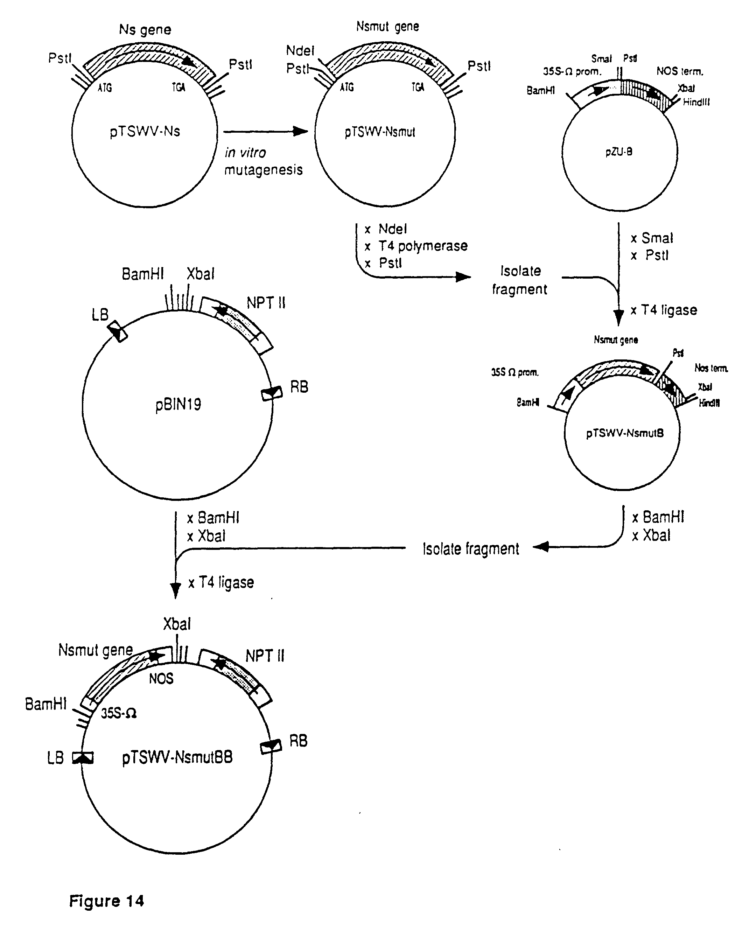

In order to create a fusion in which the ATG start codon from the NSs-protein is fused directly to the 3'-end of the TMV leader of the 35S-Ω promoter the startcodon of the NSs gene has to be mutated. Using site-directed mutagenesis, a procedure known to a person skilled in the art, the sequence 5' d(AACCATA**ATG**TCT) is mutated to 5' d(AACCAT**ATG**TCT), thereby creating a NdeI site: 5' d(CAITATG) that includes the ATG startcodon of the NSs-protein gene. The resulting plasmid is called pTSWV-NSsmut. The plasmid pTSWV-NSsmut is linearized with NdeI, followed by a treatment with T4 DNA polymerase to create blunt ends. This linearized DNA is digested with PstI and the fragment containing the mutated NSs gene is isolated and inserted into SmaI PstI linearized pZU-B resulting in the recombinant plasmid pTSWV-NsmutB. The chimeric cassette containing the 35S-Ω promoter, the mutated NSs-protein gene and the NOS terminator is excised from the plasmid pTSWV-NsmutB via a BamHI/XbaI digestion. The isolated chimeric gene cassette is then inserted into the BamHI/XbaI linearized pBIN19 to create the binary transformation vector pTSWV-NsmutBB. The resulting plasmid pTSWV-NsmutBB (figure 14) is used in plant transformation experiments using methods well known to a person skilled in the art.

### Example 9E: 5'- and 3'-termini "pan-handle" constructions in pZU-A and pZU-B

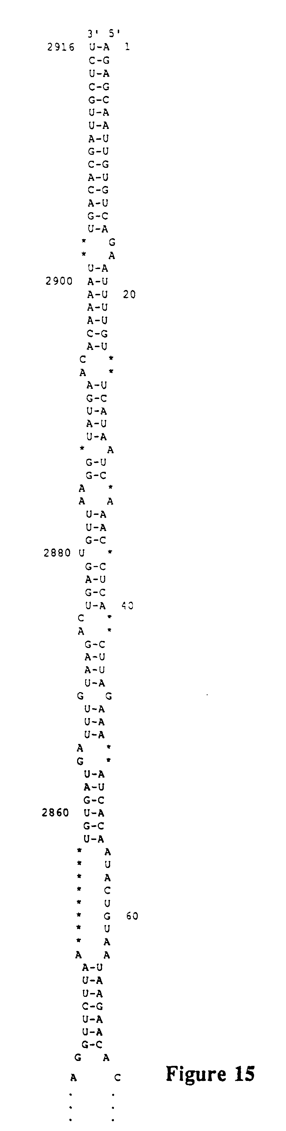

A DNA analysis programme is used to locate the "pan-handle" loop in the viral TSWV S RNA. The strongest "pan-handle" loop that is detected includes the first 70 nucleotides at the 5'-end (1 to 70) of the viral S RNA and the last 67 nucleotides at the 3'-end (2850 to 2916) of the viral S RNA (figure 15). The DNA sequence containing this "pan-handle" loop in the viral S RNA is as follows: 
1 AGAGCAATTG TGTCAGAATT TTGTTCATAA TCAAACCTCA CTTAGAAAAT
51 CACAATACTG TAATAAGAAC
 and

2850 GTTCTTAATG TGATGATTTG TAAGACTGAG TGTTAAGGTA TGAACACAAA
2900 ATTGACACGA TTGCTCT

A DNA analysis programme is used to locate the "pan-handle" loop in the viral TSWV L RNA. A strong "pan-handle" loop that is detected includes the first 80 nucleotides at the 5'end (1 to 80) of the viral L RNA and the last 80 nucleotides at the 3'-end of the viral L RNA. The DNA sequence containing this "pan-handle" loop in the viral L RNA is as follows: 
1 AGAGCAATCA GGTACAACTA AAACATATAA CCTCTCCACA GCCAGACTTT
51 ACAAATTACA TAAGAATTCC CTCCAGTGAA
 and

AAAGTGGTTC CATTTTCTAT TAATTTTTGT ATTTTCTGGA TGTTCATGTT
TGCTTAAAAT CGTTGTTACC TGATTGCTCT

These regions are synthesized on a commercial DNA synthesizer and appropriate linker sequences are added. Construction of the "pan-handle" vectors of S and L RNA results in respectively: pTSWV-termS and pTSWV-termL. Using appropriate restriction enzyme combination these fragments are inserted between the CaMV 35S promoter and the NOS terminator of pZU-A or between the 35S-Ω promoter and the NOS terminator of pZU-B yielding the chimeric cassettes: pTSWV-termSA, pTSWV-termLA, pTSWV-termSB and pTSWV-termLB. These cassettes are then transferred into the binary transformation vector pBIN19 using appropriate enzyme combinations yielding the following plasmids: pTSWV-termSAB, pTSWV-termLAB, pTSWV-termSBB and pTSWV-termLBB. Alternatively, it is possible to design "pan-handle" constucts including the 3'- and 5'-end termini that are larger as indicated above, or separated by any other DNA sequence in order to enhance the stability of the transcripts produced from these recombinant genes in plants. All "pan-handle" constructs resemble shortened tospovirus RNA, respectively TSWV RNA molecules and therefore can be regarded as defective interfering RNAs.

### Example 9F: Construction containing TSWV S RNA hairpin region in pZU-A and pZU-B.

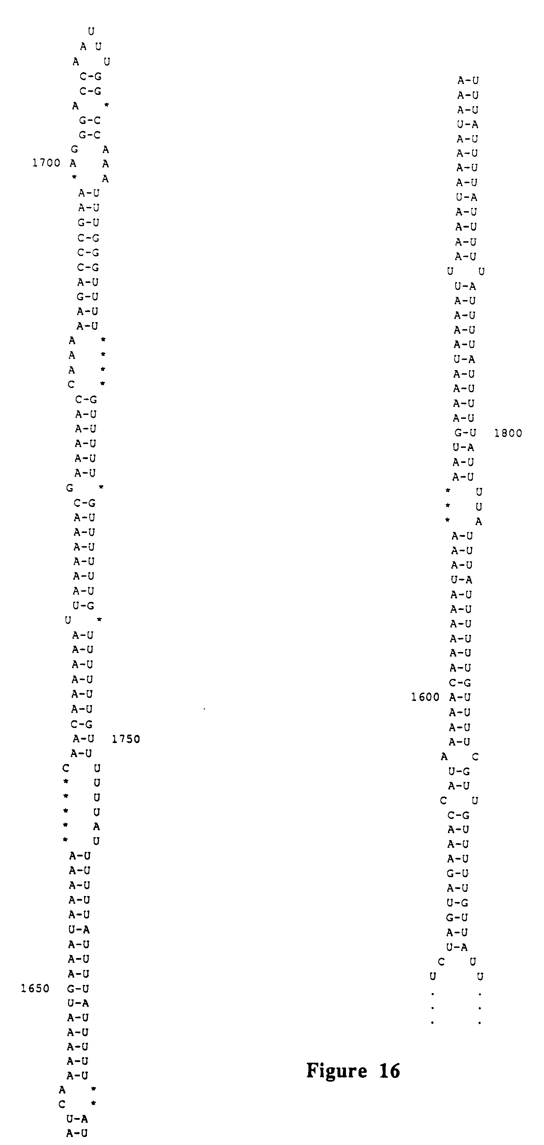

A DNA analysis programme is used to locate the hairpin loop in the viral TSWV S RNA. The strongest hairpin loop that is detected starts at nucleotide 1592 and ends at nucleotide 1834 (figure 16). The sequence containing this hairpin loop is as follows:

1 TAGTAGAAAC CATAAAAACA AAAAATAAAA ATGAAAATAA AATTAAAATA
51 AAATAAAATC AAAAAATGAA ATAAAAACAA CAAAAAATTA AAAAACGAAA
101 AACCAAAAAG ACCCGAAAGG GACCAATTTG GCCAAATTTG GGTTTTGTTT
151 TTGTTTTTTG TTTTTTGTTT TTTATTTTTT ATTTTATTTT TATTTTATTT
201 TATTTTTATT TTATTTTTAT TTTATTTATT TTTTGTTTTC GTTGTTTTTG
251 TTA

A HindIII fragment of 526 bp carrying the hairpin region is isolated from pS514. This fragment is excised from a agarose gel and subsequently treated with T4 polymerase to create blunt ends. In the following step PstI linkers are ligated to these blunt ends. After digestion with PstI the fragment is cloned in PstI linearized pZU-A, resulting in the recombinant plasmid pTSWV-HpSA. The plasmid pTSWV-HpSA is digested with HindIII and the fragment containing the chimeric gene is excised from an agarose gel and ligated into HindIII linearized pBIN19, resulting in the transformation vector pTSWV-HpSAB.

Alternatively, the HindIII fragment of pS514 is treated with T4 DNA polymerase to create blunt ends and is subsequently cloned in the SmaI site of the expression vector pZU-B, resulting in the recombinant plasmid pTSWV-HpSB. The plasmid pTSWV-HpSB is digested with HindIII and the fragment containing the chimeric gene is excised from an agarose gel and ligated into XbaI linearized pBIN19, resulting in the transformation vector pTSWV-HpSBB.

(It is clear to a person skilled in the art that also other fragments can be isolated from the cDNA clones of the TSWV S RNA containing the hairpin region as described above without interference with the function. Also, a fragment containing the hairpin region may be synthesized using a DNA-synthesizer.)

### Example 10: Transformation of binary vectors to plant material

Methods to transfer binary vectors to plant material are well established and known to a person skilled in the art. Variations in procedures exist due to for instance differences in used *Agrobacterium* strains, different sources of explant material, differences in regeneration systems depending on as well the cultivar as the plant species used.

The binary plant transformation vectors as described above are used in plant transformation experiments according to the following procedures. The constructed binary vector is transferred by tri-parental mating to an acceptor *Agrobacterium tumefaciens* strain, followed by southern analysis of the ex-conjugants for verification of proper transfer of the construct to the acceptor strain, inoculation and cocultivation of axenic explant material with the *Agrobacterium tumefaciens* strain of choice, selective killing of the *Agrobacterium tumefaciens* strain used with appropriate antibiotics, selection of transformed cells by growing on selective media containing kanamycine, transfer of tissue to shoot-inducing media, transfer of selected shoots to root inducing media, transfer of plantlets to soil, assaying for intactness of the construct by southern analyses of isolated total DNA from the transgenic plant, assaying for proper function of the inserted chimeric gene by northern analysis and/or enzyme assays and western blot analysis of proteins.

### Example 11: Expression of TSWV RNA sequences in plant cells

RNA is extracted from leaves of regenerated plants using the following protocol. Grind 200 mg leafmaterial to a fine powder in liquid nitrogen. Add 800 µl RNA extraction buffer (100 mM Tris-HCl (pH 8,0), 500 mM NaCl, 2 mM EDTA, 200 mM ß-Mercapto-ethanol, 0,4% SDS) and extract the homogenate with phenol, collect the nucleic acids by alcohol precipitation. Resuspend the nucleic acids in 0,5 ml 10 mM Tris-HCl (pH 8,0), 1 mM EDTA, add LiCl to a final concentration of 2 M, leave on ice for maximal 4 hours and collect the RNA by centrifugation. Resuspend in 400 µl 10 mM Tris-HCl (pH 8,0), 1 mM EDTA and precipitate with alcohol, finally resuspend in 50 µl 10 mM Tris-HCl (pH 8,0), 1 mM EDTA. RNAs are separated on glyoxal/agarose gels and blotted to Genescreen as described by van Grinsven *et al*. (1986). TSWV viral RNA is detected using DNA or RNA probes labeled with [³²P], [³⁵S] or by using non-radioactive labeling techniques. Based on these northern analysis, it is determined to what extent the regenerated plants express the chimeric TSWV genes.

Plants transformed with chimeric constructs containing a TSWV protein gene are also subjected to western blot analysis. Proteins are extracted from leaves of transformed plants by grinding in sample buffer according to Laemmli (1970). A 50 µg portion of protein is subjected to electrophoresis in a 12,5 % SDS-polyacrylamide gel essentially as described by Laemmli (1970). Separated proteins are transferred to nitrocellulose electrophoretically as described by Towbin *et al*. (1979). Transferred proteins are reacted with antiserum raised against purified TSWV nucleocapsids according to Towbin *et al*. (1979). Based on the results of the western analysis, it is determined that transformed plants do contain TSWV proteins encoded by the inserted chimeric genes.

### Example 12: Resistance of plants against TSWV infections

Transformed plants are grown in the greenhouse under standard quarantine conditions in order to prevent any infections by pathogens. The transformants are self-pollinated and the seeds harvested. Progeny plants are analyzed for segregation of the inserted gene and subsequently infected with TSWV by mechanical inoculation. Tissue from plants systemically infected with TSWV is ground in 5 volumes of ice-cold inoculation buffer (10 mM phosphate buffer supplemented with 1% Na₂SO₃) and rubbed in the presence of carborundum powder on the first two fully extended leafs of approximately 5 weeks old seedlings. Inoculated plants are monitored for symptom development during 3 weeks after inoculation.

Plants containing TSWV Related DNA Sequences show reduced susceptibility to TSWV infection as examplified by a delay in symptom development, whereas untransformed control plants show severe systemic TSWV symptoms within 7 days after inoculation.

### Example 13: Use of synthetic oligonucleotides for diagnostic purposes

RNA is extracted from leaves of suspected plants using the following protocol: grind 1 gram of leaf material, preferentially showing disease symptoms, in 3 ml 100 mM Tris-HCl, 50 mM EDTA, 1.5 M NaCI and 2% CTAB (pH 8.0). After grinding, 1 ml of the homogenate is subjected to chloroform extraction and incubated at 65 °C for 10 minutes. The inorganic phase is then collected and extracted with phenol/chloroform (1:1), followed by a last extraction with chloroform. The ribonucleic acids are isolated from the inorganic phase, containing the total nucleic acids, by adding LiCl to a final concentration of 2 M. The preparation is incubated at 4°C for 1 hour, after which the ribonucleic acids are collected by centrifugation. The ribonucleic acid pellet is resuspended in 25 µl 10 mM Tris-HCl, 1 mM EDTA (pH 8.0). The ribonucleic acids are recovered by standard alcohol precipitation. The ribonucleic acid pellet is resuspended in 25 µl 10 mM Tris-HCI, 1 mM EDTA (pH 8.0).

1 µl of the purified ribonucleic acids is spotted on a nylon blotting membrane (e.g. Hybond-N, Amersham UK). The presence of TSWV in the plant is detected by standard hybridization, using any part or parts of the sequence isolated from virions or preferentially by designing synthetic oligomers on the basis of disclosed sequence information as a probe. (Alternatively, *in vitro* transcripts of regions of the TSWV genome are used to detect TSWV Related RNA Sequences in diseased plants.) A diseased plant is diagnosed by the occurrence of hybridization at the dot containing RNA material from the diseased plant.

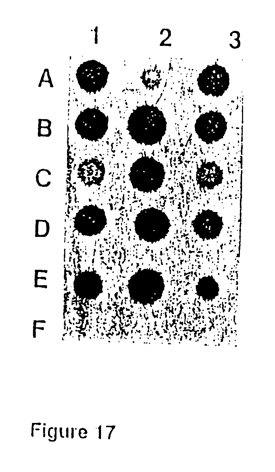

Using procedures as described herein RNA is isolated from 12 pepper plants (A1 - D3) suspected to be virus infected selected from a field. Similarly, RNA is extracted from 3 TSWV inoculated tobacco plants (E1 - E3) and from 3 non-inoculated tobacco plants (F1 - F3). From each plant an RNA sample of 1 µl is spotted onto a Hybond membrane. This filter is hybridized under standard conditions with an *in vitro* transcript synthesized from the cDNA clone pS614 using T3 polymerase.and α-[³²P]UTP as an radioactive label. Control non-infected plants (F1 - F3) do not show a signal, control TSWV infected plants (E1 - E3) do show a strong signal, whereas suspected pepper plants all show signals ranging in intensity from weak to strong (see figure 17).

### References

Abel PP, Nelson RS, De B, Hoffmann N, Rogers SG, Fraley RT and Beachy RN (1986) Delay of disease development in transgenic plants that express the tobacco mosaic virus coat protein gene. Science 232, 738-743.
Ausubel FM, Brent R, Kingston RE, Moore DD, Seidman JG, Smith JA, Struhl K (1987) Current protocols in molecular biology. Green Publishing Associates and Wiley Intersciences, New York, Chichester, Brisbane, Toronto and Singapore.
Beachy RN, Fraley RT and Rogers SG, EP 0 223 452: Protection of plants against viral infection. Monsanto Company & Washington University.
Bevan M (1984) Binary *Agrobacterium* vectors for plant transformation. Nucl Acids Res 12, 8711-8721.
Clerx-Van Haaster CM and Bishop DHL (1980) Analysis of the 3'-terninal RNA sequences of Snowshoe hare and Lacrosse Bunyaviruses. Virolology 105, 564-574.
Clerx-Van Haaster CM, Clerx JPM, Ushijima H, Akashi H, Fuller F and Bishop DHL (1982) Analysis of the 3'-terminal RNA sequences of Bunyaviruses and Nairoviruses (Bunyaviridae): evidence of end sequence generic differences within the virus family. J Gen Virol 61, 289-292.
Figurski D, Helinski DR (1979) Replication of an origin containing a derivative of plasmid RK₂ dependent on a plasmid function provided in *trans*. Proc Natl Acad Sci USA 76: 1648-1652
Franck A, Guilley H, Jonard G, Richards K and Hirth L (1980) Cell 21, 285-294.
Grunstein JM and Hogness DS (1975) Colony hybridization: a method for the isolation of cloned DNAs that contain a specific gene. Proc Natl Acad Sci USA 72, 3961-3965.
Gubler U, Hoffman BJ (11983) A simple and very efficient method for generating cDNA libraries. Gene 25, 263-269.
Hoekema A, Hirsch PR, Hooykaas PJJ, Schilperoort RA (1983) A binary vector system based on separation of vir- and T-region of the *Agrobacterium tumefaciens* Ti-plasmid. Nature 303: 179-180
Huynh TH, Young RA and Davis RW (1985) Construction and screening cDNA libraries in lambda gt10 and lambda gt11. In: DNA cloning techniques: a practical approach. (Glover D, ed.) IRL Press, Oxford, pp. 49-78.
Laemmli UK (1970) Cleavage of structural proteins during the assembly of the head of bacteriophage T4. Nature 244, 29-30.
Maniatis T, Fritsch EF, Sambrook J (1982) Molecular cloning, a laboratory manual. Cold Spring Harbor Laboratory, New York
Murashige T, Skoog F (1962) A revised medium for rapid growth and bio-assays with tobacco tissue cultures. Physiol Plant 15: 473-497
Tas PWL, Boerjan ML and Peters D (1977) The structural proteins of tomato spotted wilt virus. J Gen Virol 36, 81-91.
Towbin H, Staehelin T and Gordon J (1979) Electrophoretic transfer of proteins from polyacrylamide gels to nitrocellulose: procedure and some applications. Proc Natl Acad Sci USA 76, 4350-4354.
Van Grinsven MQJM, Gielen JJL, Zethof JLA, Nijkamp HJJ and Kool AJ (1986) Transcriptional and post-transcriptional regulation of chloroplast gene expression in *Petunia hybrida*. Theor Appl Gen 73, 94-101.
Verkleij FN and Peters D (1983) Characterization of a defective form of tomato spotted wilt virus. J Gen Virol 64, 677-686.
Wieslander R (1979) A simple method to recover intact high molecular weight RNA and DNA after electrophoretic separation in low gelling temperature agarose gels. Anal Biochem 98, 305-309.
Yanish-Perron C; Vieira J and Messing J (1985) Improved M13 phage cloning vectors and host strains: nucleotide sequence of the M13mp18 and pUC19 vectors. Gene 33, 103-119.

## Claims

1. Recombinant DNA constructs comprising a DNA sequence under expression control of a promoter and a terminator functioning in plants, which DNA sequence encodes:
(a) an RNA sequence selected from the group consisting of the following tospoviral sequences:
(i) the S RNA nucleotide sequence from 1 to 2915 depicted in Figure 4;
(ii) the S RNA nucleotide sequence from 2763 to 1987 depicted in Figure 4; or
(b) an RNA sequence according to (a) encoding for a tospovirus protein in which one or more codons have been replaced by their synonyms (i.e. codons corresponding to the same amino acid or termination signal), or part thereof comprising at least 20 nucleotides, or an RNA sequence homologous thereto and comprising at least 20 nucleotides wherein a number of nucleotides have been deleted and/or added but which is still capable of hybridization to a nucleotide sequence complementary to an RNA sequence according to (a) under appropriate hybridization conditions including incubation for 16 hours at 42°C, in a buffer system comprising 5x standard saline citrate, 0.5% sodium dodecylsulfate, 5x Denhardt's solution, 50% formamide and 100µg/ml carrier DNA, followed by washing 3 times with a buffer comprising 1x SSC and 0.1% SDS at 65°C for approximately 1 hour each time; or
(c) an RNA sequence complementary to an RNA sequence according to (a) or (b).

2. The DNA construct of claims 1, wherein the promoter is a viral, fungal, bacterial, animal or plant derived promoter functioning in plant cells.

3. The DNA construct of any one of the preceding claims, wherein the terminator is a viral, fungal, bacterial, animal or plant derived terminator functioning in plant cells.

4. A process of preparing a differentiated plant or undifferentiated plant material comprising in its genome a DNA construct according to any one of claims 1 to 3 which comprises:
a) inserting into the genome of a plant cell a DNA construct of claim 1,
b) obtaining transformed cells,
c) regeneration of genetically transformed plants from the transformed cells.

5. A differentiated plant or undifferentiated plant material comprising in its genome a DNA construct according to claims 1-3.

6. Undifferentiated plant material according to claim 5 selected from the group consisting of protoplasts, plant cells, seeds and plantlets that under appropriate conditions can develop into mature plants, the progeny thereof and parts thereof.

## Patentansprüche

1. Rekombinante DNA Konstrukte, die eine DNA Sequenz unter einer Expressionskontrolle eines Promotors und eines Terminators enthalten, die in Pflanzen funktionsfähig sind, wobei die DNA Sequenz folgendes kodiert:
(a) eine RNA Sequenz, ausgewählt aus der Gruppe, die besteht aus den folgenden tospoviralen Sequenzen
(i) S RNA Nukleotidsequenz von 1 bis 2915, die in Figur 4 gezeigt ist,
(ii) S RNA Nuleotidsequenz von 2763 bis 1987, die in Figur 4 gezeigt ist, oder
(b) eine RNA Sequenz gemäß (a), die kodiert für ein Tospovirusprotein, worin ein oder mehrere Kodons durch ihre Synonyme ersetzt wurden (das heißt Kodons, die derselben Aminosäure oder dem Terminationssignal entsprechen) oder ein Teil hiervon, der zumindest 20 Nukleotide umfaßt, oder eine RNA Sequenz, die hierzu homolog ist und zumindest 20 Nukleotide umfaßt, worin mehrere Nukleotide deletiert und/oder hinzugefügt wurden, die aber immer noch zur Hybridisierung mit einer Nukleotidsequenz, die zu einer RNA Sequenz gemäß (a) komplementär ist unter geeigneten Hybridisierungsbedingungen fähig ist, einschließlich einer Inkubation für 16 Stunden bei 42°C in einem Puffersystem, das aus 5x Standardkochsalzcitrat, 0,5 % Natriumdodecylsulfat, 5x Denhardt's Lösung, 50 % Formamid und 100 µg/ml Träger DNA besteht, gefolgt von einem dreimaligen Waschen mit einem Puffer, der aus 1x SSC und 0,1 % SDS besteht, bei 65°C für jeweils etwa 1 Stunde, oder
(c) eine RNA Sequenz, die zu einer RNA Sequenz gemäß (a) oder (b) komplementär ist.

2. DNA Konstrukt nach Anspruch 1, worin der Promotor ein in Pflanzenzellen funktionsfähiger Promotor aus einem Virus, einem Pilz, einem Bakterium, einem Tier oder einer Pflanze ist.

3. DNA Konstrukt nach einem der vorangehenden Ansprüche, worin der Terminator ein in Pflanzenzellen funktionsfähiger Terminator aus einem Virus, einem Pilz, einem Bakterium, einem Tier oder einer Pflanze ist.

4. Verfahren zur Herstellung einer differenzierten Pflanze oder eines undifferenzierten Pflanzenmaterials, das im Genom ein DNA Konstrukt nach einem der Ansprüche 1 bis 4 enthält, das umfaßt:
a) Insertion eines DNA Konstrukts nach Anspruch 1 in das Genom einer Pflanzenzelle,
b) Erhalten von transformierten Zellen,
c) Regeneration von genetisch transformierten Pflanzen aus den transformierten Zellen.

5. Differenzierte Pflanze oder undifferenziertes Pflanzenmaterial, das im Genom ein DNA Konstrukt nach den Ansprüchen 1-3 enthält.

6. Undifferentiertes Pflanzenmaterial nach Anspruch 5, ausgewählt aus der Gruppe, die besteht aus Protoplasten, Pflanzenzellen, Samen und Pflänzchen, die sich unter geeigneten Bedingungen zu reifen Pflanzen entwickeln können, die Nachkommen hiervon und Teile hiervon.

## Revendications

1. Produits de construction d'ADN recombinant, comprenant une séquence d'ADN sous contrôle d'expression d'un promoteur et d'un terminateur fonctionnant chez des plantes, laquelle séquence d'ADN code pour :
(a) une séquence d'ARN choisie dans l'ensemble constitué par les séquences tospovirales suivantes :
(I) la séquence nucléotidique d'ARN S de 1 à 2915 représentée sur la figure 4 ;
(II) la séquence nucléotidique d'ARN S de 2763 à 1987 représentée sur la figure 4 ; ou
(b) une séquence d'ARN selon (a) codant pour une protéine de tospovirus, dans laquelle un ou plusieurs codons ont été remplacés par leurs synonymes (c'est-à-dire des codons correspondant au même aminoacide ou signal de terminaison), ou une partie de celle-ci comprenant au moins 20 nucléotides, ou une séquence d'ARN homologue de celle-ci et comprenant au moins 20 nucléotides, dans laquelle un certain nombre de nucléotides ont été supprimés et/ou ajoutés mais qui est encore capable d'hybridation avec une séquence nucléotidique complémentaire d'une séquence d'ARN selon (a) dans des conditions d'hybridation appropriées comprenant une incubation pendant 16 heures à 42°C, dans un système tampon comprenant une solution standard de chlorure de sodium au citrate (SSC) 5x, 0,5 % de dodécylsulfate de sodium, solution de Denhardt 5x, 50 % de formamide et 100 µg/ml d'ADN vecteur, suivie d'un lavage à trois reprises avec un tampon comprenant SSC 1x et 0,1 % de SDS à 65°C pendant environ 1 heure chaque fois ; ou
(c) une séquence d'ARN complémentaire d'une séquence d'ARN selon (a) ou (b).

2. Produit de construction d'ADN de la revendication 1, dans lequel le promoteur est un promoteur viral, fongique, bactérien ou issu d'un animal ou d'une plante, fonctionnant dans des cellules végétales.

3. Produit de construction d'ADN de l'une quelconque des revendications précédentes, dans lequel le terminateur est un terminateur viral, fongique, bactérien ou issu d'un animal ou d'une plante, fonctionnant dans des cellules végétales.

4. Procédé de production d'une plante différenciée ou d'un matériel végétal non différencié, comprenant dans son génome un produit de construction d'ADN selon l'une quelconque des revendications 1 à 4, qui comprend :
a) l'insertion d'un produit de construction d'ADN de la revendication 1 dans le génome d'une cellule végétale,
b) l'obtention de cellules transformées,
c) la régénération de plantes génétiquement modifiées à partir des cellules transformées.

5. Plante différenciée ou matériel végétal non différencié, comprenant dans son génome un produit de construction d'ADN selon les revendications 1 à 3.

6. Matériel végétal non différencié selon la revendication 5, choisi dans l'ensemble constitué par des protoplastes, des cellules végétales, des graines et des plantules, gui, dans des conditions appropriées, peuvent se développer en plantes matures, descendance d'un tel matériel et parties de celui-ci.
